(19) Europäisches Patentamt
European Patent Office
Office européen des brevets

(11) Publication number: 0 182 165 B1

(12) # EUROPEAN PATENT SPECIFICATION

(45) Date of publication of patent specification: 16.08.89

(21) Application number: 85113913.9

(22) Date of filing: 31.10.85

(51) Int. Cl.[4]: C 07 D 471/04, C 07 D 487/04, A 61 K 31/435, A 61 K 31/50 // C07D333/36, C07D409/12 ,(C07D401/04, 231:00, 221:00),(C07D487/04, 237:00, 231:00)

(54) **Thienyl condensed-pyrazole derivatives.**

(30) Priority: 05.11.84 JP 233511/84

(43) Date of publication of application: 28.05.86 Bulletin 86/22

(45) Publication of the grant of the patent: 16.08.89 Bulletin 89/33

(84) Designated Contracting States: CH DE FR IT LI SE

(56) References cited:
EP-A-0 022 078
FR-A-2 549 833
GB-A-2 131 801
US-A-4 312 870

(73) Proprietor: SHIONOGI & CO., LTD.
1-8, Doshomachi 3-chome Chuo-ku
Osaka 541 (JP)

(72) Inventor: Takada, Susumu
4-6-78, Midoridai
Kawanishi-shi Hyogo (JP)
Inventor: Sasatani, Takashi
2182-9, Mise-cho
Kashihara-shi Nara (JP)
Inventor: Shindo, Hirohisa
1-18-3, Higashinoda-cho Miyakojima-ku
Osaka-shi Osaka (JP)
Inventor: Matsushita, Akira
2-3-22, Fukaeminamimachi Higashinada-ku
Kobe-shi Hyogo (JP)
Inventor: Eigyo, Masami
2-10-7, Shikanodainishi
Ikoma-shi Nara (JP)

(74) Representative: Bruin, Cornelis Willem et al
Octrooibureau Arnold & Siedsma Isartorplatz 5
D-8000 München 2 (DE)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 182 165 B1

**Description**

This invention relates to a new group of pyrazoloquinoline derivatives which distinguish themselves by having a potent psychotropic activity.

It is known that several 2-substituted pyrazolo[4,3-c]quinolin-3-one derivatives have psychotropic activities in consequence of a strong binding affinity to benzodiazepine receptors. Thus, anxiolytic and antidepressive activities have been described for pyrazolo[4,3-c]quinolin-3-one derivatives which bear a phenyl group in 2-position (US—A—4 312 870) whereas anxiolytic, anticonvulsive and benzodiazepine antagonistic activities have been described for pyrazolo[4,3-c]quinolin-3-one derivatives which bear a quinolyl, isoquinolyl, pyrimidyl or thiazolyl group in 2-position (GB—A—2 131 801).

In this connection, it should be noted that compounds binding to benzodiazepine receptors can be classified into three separate groups, namely: (a) agonists which have utility as minor tranquilizers or as anticonvulsants, (b) antagonists which can be used for treating benzodiazepine intoxications and accidental supernumerary uptakes, and (c) inverse agonists which can be expected to act as vigilance-enhancing compounds. The derivtives described in US—A—4 312 870 apparently belong to group (a) whereas the derivatives described in GB—A—2 131 801 will belong to group (a) and/or group (b).

During further research, it has now been found that pyrazolo[4,3-c]quinolin-3-one derivatives bearing a thienyl group in 2-position also have a high affinity to benzodiazepine receptors and that such derivatives, in distinction to the derivatives of US—A—4 312 870 and GB—A—2 131 801, are primarily of the inverse agonist type (group c) although agonist and antagonist properties will also be present. This means that the new derivatives can be used with advantage in pharmaceutical compositions having psychotropic activities and with special advantage in compositions aimed at having a vigilance-enhancing effect. The invention is based on these findings.

The pyrazolo[4,3-c]quinolin-3-one derivatives derivatives of the invention can be represented by the following formula (I):

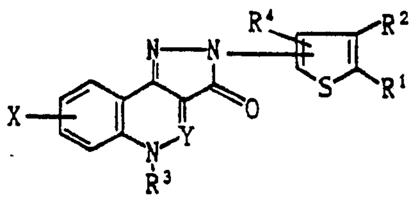

(I)

wherein $R^1$ and $R^2$ each are hydrogen, $(C_1—C_5)$alkyl, $(C_2—C_6)$alkoxycarbonyl, carboxyl, halogen, nitro or trifluoromethyl, or $R^1$ and $R^2$ when taken together are $(C_3—C_4)$ alkylene; $R^3$ is hydrogen, $(C_1—C_5)$alkyl, $(C_1—C_5)$alkanoyl or $(C_1—C_5$alkylsulfonyl; $R^4$ is hydrogen, $(C_2—C_6)$alkoxycarbonyl, carboxyl, or halogen; X is hyrogen, $(C_1—C_5)$alkyl, $(C_1—C_5)$alkoxy, halogen or hydroxyl; and Y is methine or nitrogen. Salts of these derivatives are included in the definition.

Thus, the present invention relates to pyrazolo[4,3-c]quinolin-3-one derivatives of formula (I) and their salts as well as to the use of such derivatives for preparing pharmaceutical compositions having psychotropic activities.

The biological activities of the derivatives in question are illustrated by the following experiments wherein the number of each tested compound of the invention corresponds to the number used in the Examples given later on.

Experiment 1

Affinity to benzodiazepine receptors

This test was carried out by the modified method of Moeler & Okada, Science, *198* 849—851 (1977).

A receptor preparation was provided from the cerebral cortex of Wistar rats (male, 11 to 13 weeks age). The inhibitory action of the test compound on the specific binding of tritium-labeled diazepam to benzodiazepine receptors was evaluated as follows. 2nM tritium-labeled diazepam and an aqueous solution of the test compound at 5 or 6 concentrations were incubated with the receptor preparation at 0°C for 60 minutes. The 50% inhibitory concentration ($IC_{50}$) was measured by means of a concentration-response curve.

The inhibitory constant (Ki) was calculated according to the following equation, in which Kd is the dissociation constant of the tritium-labeled diazepam and L is the concentration of the labeled ligand.

$$Ki = \frac{IC_{50}}{1 + L/Kd}$$

The results are shown in the following table.

| Compd. No. | Ki (nM) | Compd. No. | Ki (nM) |
|---|---|---|---|
| $Ic_1$-1 | 0.35 | $Ic_2$-1 | 0.29 |
| $Ic_1$-2 | 1.51 | $Ic_2$-2 | 0.45 |
| $Ic_1$-3 | 0.63 | $Ic_2$-4 | 0.30 |
| $Ic_1$-5 | 1.53 | $Ic_2$-5 | 0.39 |
| $Ic_1$-6 | 0.22 | $Ic_2$-6 | 0.58 |
| $Ic_1$-7 | 0.17 | $Ic_2$-7 | 0.17 |
| $Ic_1$-8 | 0.31 | $Ic_2$-8 | 1.24 |
| $Ic_1$-9 | 0.14 | $Ic_2$-9 | 0.96 |
| $Ic_1$-10 | 1.44 | | |
| $Ic_1$-11 | 1.11 | | |
| $Ic_1$-12 | 0.38 | | |

Experiment 2

Inverse agonist activity

The test compound was administered orally to a group of 8—16 male mice, 1 hour in advance to a subcutaneous administration of 75 mg/kg of pentylenetetrazol (a subconvulsive dose). The dose ($ED_{50}$) at which 50% of mice died, was calculated by the probit method.

| Compd. | $ED_{50}$ (mg/kg) | Note |
|---|---|---|
| $Ic_2$-2 | 1.67 | Subject matter of the invention |
| Control 1 | > 200.0 | US-A-4 312 870 |
| Control 2 | 87.02 | GB-A-2 131 801 |

*Notes to Table*

Control 1: 2-Phenyl-2,5-dihydro-3H-pyrazolo[4,3-c]quinolin-3-one
Control 2: 2-(2-Thiazolyl)-2,5-dihydro-3H-pyrazolo[4,3-c]quinolin-3-one

The derivatives of formula (I) can be prepared according to three different methods, indicated as methods, A, B and C. Method A is illustrated by the following reaction scheme wherein A is a leaving group such as methoxy, ethoxy, n-propoxy, isopropoxy, n-butoxy, isobutoxy, sec. butoxy or tert.butoxy; Hal is halogen; R is alkyl; and $R^1$, $R^2$, X and Y have the same meanings as defined for formula (I).

Method A

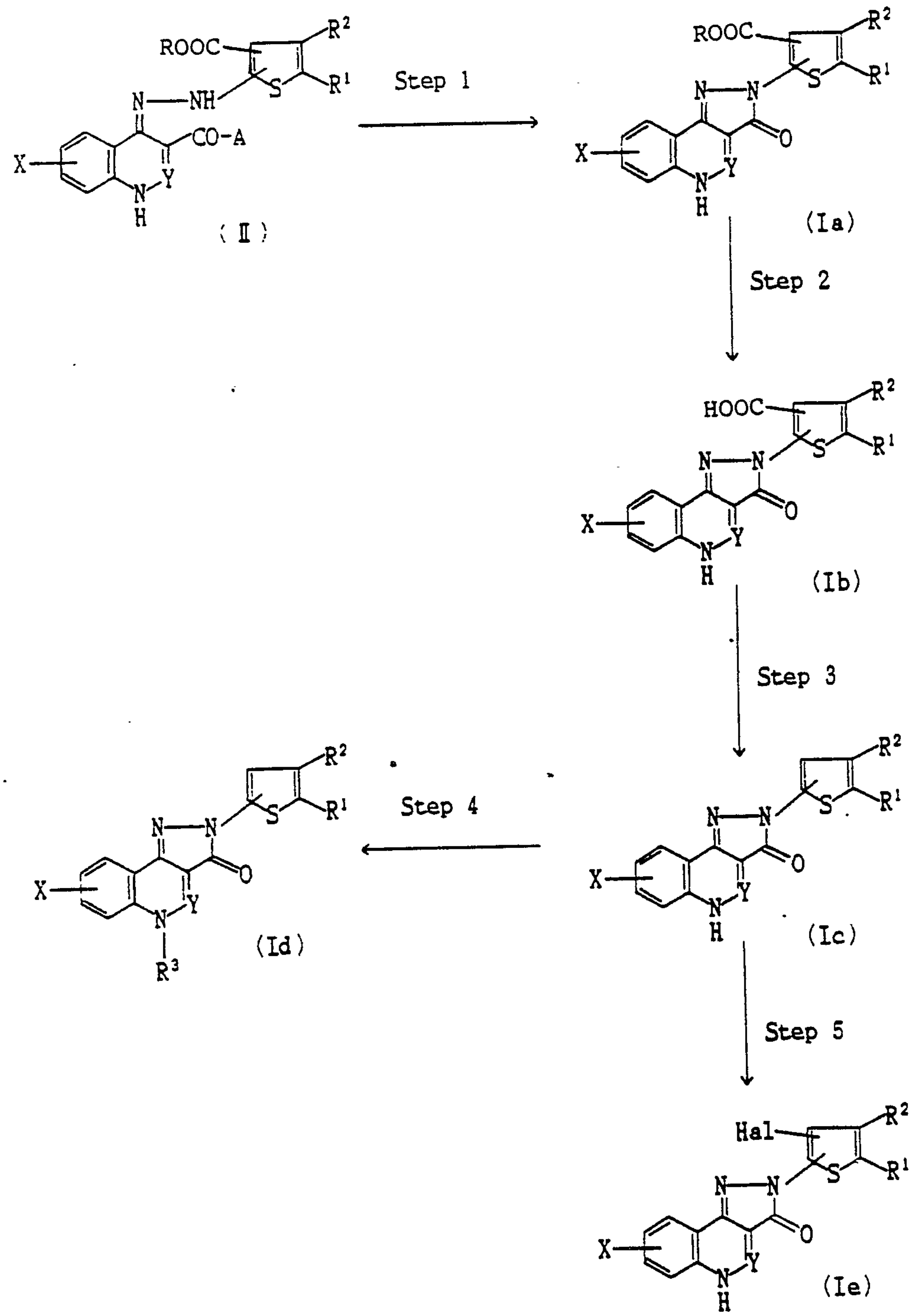

(II)
(Ia)
(Ib)
(Ic)
(Id)
(Ie)

Steps 1 to 5 of method A will now be explained in more detail.

Step 1

In this step, compound (Ia) is prepared by cyclization of a starting material (II). The reaction can be carried out in a solvent such as an alkanol (e.g. methanol, ethanol, isopropanol), a halogenohydrocarbon (e.g. dichloromethane, dichloroethane, chloroform, carbon tetrachloride), an aromatic solvent (e.g. benzene, toluene, xylene), or dimethylformamide; at a temperature of about 10°- about 100°C, and if required in the presence of an inorganic base such as an alkali metal hydroxide (e.g. potassium hydroxide, sodium hydroxide, lithium hydroxide), an alkali metal carbonate (e.g. potassium carbonate, sodium carbonate) or an alkali metal bicarbonate (e.g. potassium bicarbonate, sodium bicarbonate); an organic base such as triethylamine, pyridine, picoline, quinoline, piperidine, pyrrolidine or N-methylmorpholine, an inorganic acid such as hydrochloric acid, sulfuric acid or phosphoric acid; or an organic acid such as acetic acid, trifluoroacetic acid, or toluenesulfonic acid.

The base or acid may be selected in accordance with the properties of the substituents at the thienyl group of the starting material (II) and the properties of the leaving group A. The reaction is preferably carried out at a temperature of about 10° — about 30°C, if a base or an acid is used.

The reaction may be accelerated under inert gas flow such as nitrogen or argon.

The starting material (II) is, for example, provided according to the following method:

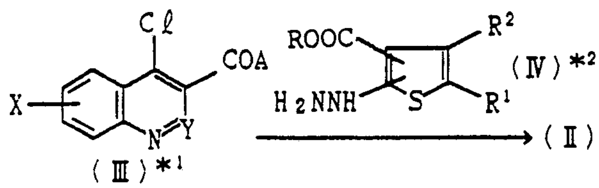

wherein A, R, $R^1$, $R^2$, X and Y have the same meanings as in the scheme of Method A.

*1 J. Am. Chem. Soc., *68*, 1264 (1964)

J. Org. Chem., *18*, 55 (1953)

*2 J. Prakt. Chem., *316*, 878 (1974)

Step 2

In this step, compound (Ia) is hydrolized to give compound (Ib). The reaction can be conducted in a conventional manner, for example by treating the compound (Ia) with an inorganic base in a solvent. The solvent may be selected from water; alkanols (e.g. methanol, ethanol, isopropanol); halogenohydrocarbons (e.g. dichloromethane, dichloroethane, chloroform, carbon tetrachloride); ether (e.g. ether, tetrahydrofuran, dioxane); dimethylformamide; dimethylsulfoxide; or mixtures thereof. Further, an alkalimetal hydroxide, alkalimetal carbonate, or alkalimetal bicarbonate may be employed as an inorganic base. The reaction is performed at a temperature of about 30° — about 120°C and preferably from about 50° — about 80°C, under an inert gas flow such as nitrogen or argon in the same manner as in Step 1. Step 2 can be carried out continuously after Step 1.

Step 3

The decarboxylation of the thiophene carboxylic acid (Ib) is performed in an organic solvent such as quinoline or isoquinoline, at a temperature of about 150°C — about 250°C, and if necessary, in the presence of a catalyst such as copper powder or chromous acid-copper ($CuO.Cr_2O_3$).

The reaction can also be conducted as follows. First, compound (Ib) is converted into its alkalimetal salt by treatment with an alkalimetal hydroxide, and then the alkalimetal salt of (Ib) is heated at about 150° — about 250°C in the presence of a base (e.g. calcium oxide-sodium hydroxide).

Step 4

The objective compound (Id) is prepared by introduction of an alkanoyl group to compound (IC) or by alkylation of (Ic). The reaction is conducted with an alkylating agent or an alkanoyl group introducing agent in an appropriate solvent in the presence of an alkalimetal hydride such as sodium hydride or potassium hydride. The reaction is carried out at a temperture of 30°—120°C. An alkyl halogenide (e.g. methyl bromide, ethyl iodide, propyl chloride) or a dialkyl sulfate (e.g. dimethyl sulfate, diethyl sulfate) may be used as an alkylating agent, whereas an alkanoyl halide (e.g. acetyl chloride, butyryl bromide) or an alkanoic acid anhydride (e.g. acetic anhydride, propionic anhydride) can be used as an alkanoyl group introducing agent. Further, tetrahydrofuran, dioxane, diglyme and dimethylformamide are preferred solvents.

Step 5

Compound (Ie) is prepared by halogenation of compound (Ic). The halogenation can be performed by reacting the compound (Ic) with a halogen such as fluorine, chlorine, bromine, iodine in a appropriate solvent in a conventional manner. Halogenohydrocarbons such as dichloromethane, dichloroethane,

chloroform or carbon tetrachloride may be employed as a solvent. The reaction is accomplished at a temperature from room temperature to refluxing temperature within a period of several hours to several tens of hours.

The halogenation can also be performed by means of N-bromosuccinimide, N-chlorosuccinimide or sulfuryl chloride.

Compound (Ic) may be also prepared according to Method B, which is illustrated by the following reaction scheme:

Method B

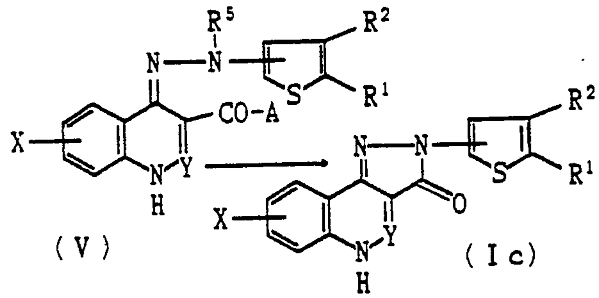

(V) (Ic)

wherein A, $R^1$, $R^2$, X and Y have the same meanings as defined for the reaction scheme of method A; and $R^5$ is alkoxy carbonyl, acetyl or trifluoroacetyl.

Method B is carried out by reacting compound (V) with an acid at a temperature between room temperature and about 100°C in an appropriate solvent, if required with addition of a base. The acid is preferably a strong acid such as trifluoroacetic acetic acid or hydrobromic acid-acetic acid. The base may be selected suitably from inorganic bases such as alkalimetal hydroxides (e.g. potassium hydroxide, sodium hydroxide, lithium hydroxide); alkalimetal carbonates (e.g. potassium carbonate, sodium carbonate); and alkalimetal bicarbonates (e.g. potassium bicarbonate, sodium bicarbonate); and organic bases such as triethylamine, pyridine, picoline, quinoline, piperidine, pyrrolidine or N-methylmorpholine. The solvent used in the reaction is exemplified by alkanols (e.g. methanol, ethanol, isopropanol); halogenohydrocarbons (e.g. dichloromethane, dichloroethane, chloroform, carbon tetrachloride); and ethers (e.g. dibutyl ether, tetrahydrofuran).

The reaction is accomplished in a period of several tens of minutes to several hours and leads to compound (Ic).

Compound (Id) and compound (Ie) are prepared from compound (Ic) in the same manner as in Steps 4 and 5 of Method A.

The objective compound (I) wherein $R^1$ is hydrogen can be prepared by Method C, which is illustrated by the following reaction scheme, wherein A, $R^2$, $R^4$, X and Y have the same meanings as in the reaction scheme of Method A, and $R^1$ is alkyl.

Method C

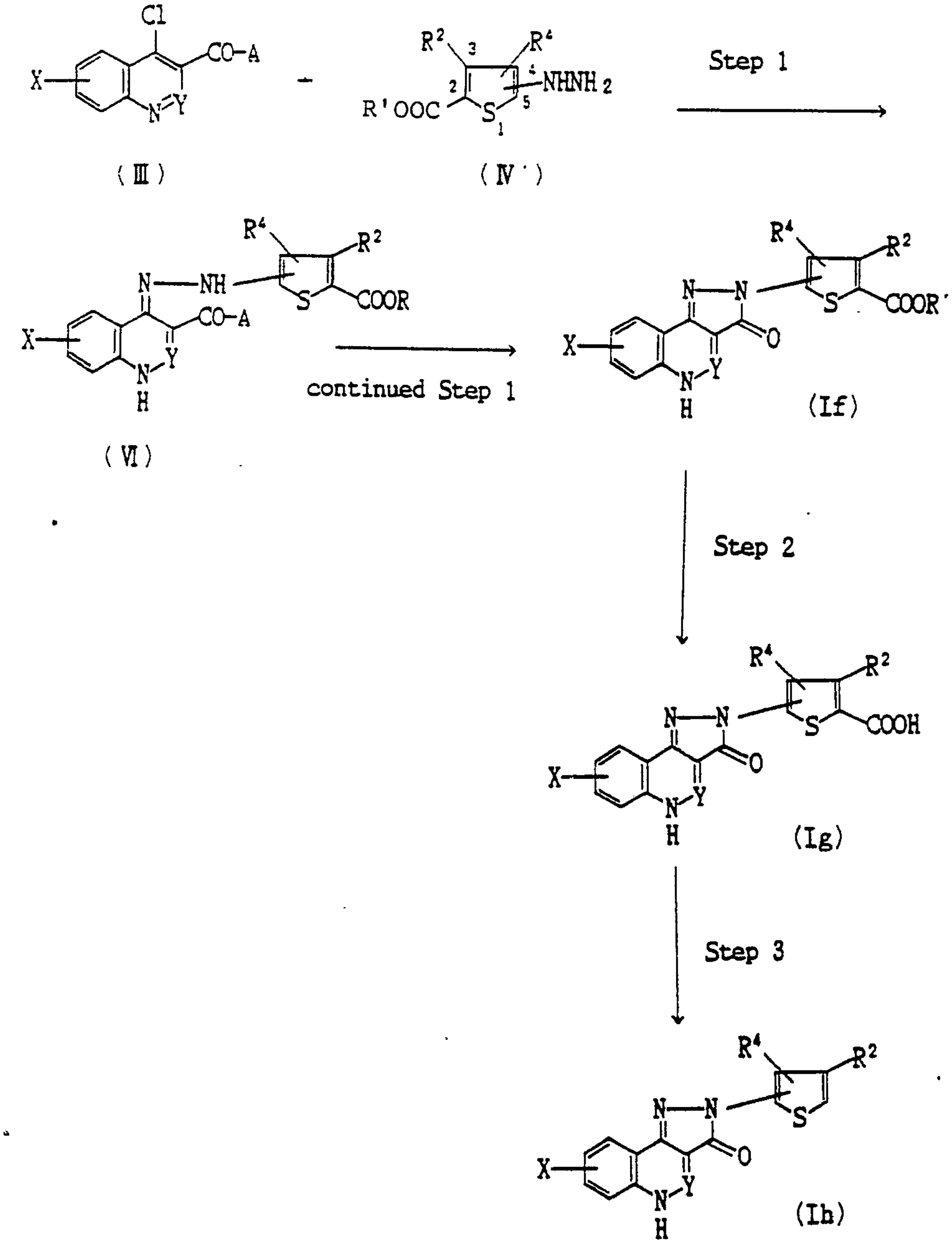


(III) (IV') (VI) (If) (Ig) (Ih)

Steps 1 to 3 of Method C can be conducted as follows.

Step 1

In this step, a compound (IV') is used in which the thiophene nucleus is protected at its 2-position. For example, a compound (IV') protected by alkoxycarbonyl (R'OOC—) in 2-position or an acid addition salt such as a hydrochloride thereof is allowed to react with compound (III) in an appropriate solvent at a temperature of about 10° — about 100°C, if required with addition of a base or an acid. The solvent includes alkanols (e.g. methanol, ethanol, isopropanol), halogenohydrocarbons (e.g. dichloromethane, dichloroethane, chloroform, carbon tetrachloride); aromatic solvents (e.g. benzene, toluene, xylene); and dimethylformamide. The base can be selected from inorganic bases such as an alkalimetal hydroxide, alkalimetal carbonate, alkalimetal bicarbonate; and organic bases such as triethylamine, pyridine, picoline, quinoline, piperidine, pyrrolidine or N-methylmorpholine. Further, inorganic acids such as hydrochloric acid, sulfuric acid or phosphoric acid, and organic acids such as acetic acid, trifluoroacetic acid and toluenesulfonic acid, can be employed as an acid.

In this step, it seems that compound (VI) is produced as an intermediate, although the cyclizised compound (If) has been obtained directly as a product.

Step 2

In this step, compound (Ig) is prepared by hydrolysis of compound (If).

The reaction may be conducted in the same manner as in Step 2 of Method A, for example by treating compound (If) with an organic base in an appropriate solvent.

The Solvent includes water; alkanols (e.g. methanol, ethanol, isopropanol); halogenohydrocarbons (e.g. dichloromethane, dichloroethane, chloroform, carbon tetrachloride); ethers (e.g. ether, tetrahydrofuran, dioxane); dimethylformamide, dimethylsulfoxide, and mixtures thereof. An alkalimetal hydroxide, alkalimetal carbonate or alkalimetal bicarbonate may be employed as an inorganic base. The reaction may be carried out at a temperature of about 30° — about 120°C and preferably about 50° — about 80°C, under an inert gas flow such as nitrogen or argon.

Step 3

Compound (Ih) can be prepared by decarboxylation of compound (Ig); the reaction may be carried out in the same manner as in Step 3 of Method A.

The reaction can be carried out in an solvent such as quinoline or insoquinoline at a temperature of about 150° — about 250°C and, if necessary, in the presence of a catalyst such as copper powder or chromous acid-copper ($CuO.Cr_2O_3$).

The reaction can also be performed as follows. First, compound (Ig) is converted into its alkalimetal salt by treatment with an alkalimetal hydroxide, and then the alkali-metal salt of (Ig) is heated at about 150° — about 250°C in the presence of a base (e.g. calcium oxide-sodium hydroxide).

In Method C, Steps 1 to 3 can be carried out continuously, or the product in each step can be isolated.

Compound (Ih) can be subjected to alkylation or introduction of an alkanoyl group in the same manner as in Step 4 of Method A. Further, compound (Ih) can be halogenated in the same manner as in Step 5 of Method A.

The objective compound (I) can also be prepared by cyclisation of a 4-[2(3)-thienylhydrazono]-3-carboxylic acid ester. The ester is produced from a compound in which the substituent on the thiophene nucleus of the corresponding compound (IV) or (IV') (i.e. alkoxycarbonyl ROOC—, R'OOC—) is replaced by an electron-withdrawing group such as a nitro group.

The objective compounds (I) can be converted into acid-addition salts with the aid of inorganic or organic acids, if desired. Suitable inorganic acids are hydrochloric acid, hydrobromic acid, sulfuric acid, phosphoric acid, nitric acid; suitable organic acids are acetic acid, methanesulfonic acid, succinic acid, maleic acid, tartaric acid, benzoic acid.

The compounds (I) can be converted into alkalimetal salts such as sodium, potassium, or lithium salts.

The objective compounds (I) wherein $R^3$ is hydrogen can exist in two tautomeric forms, as shown below:

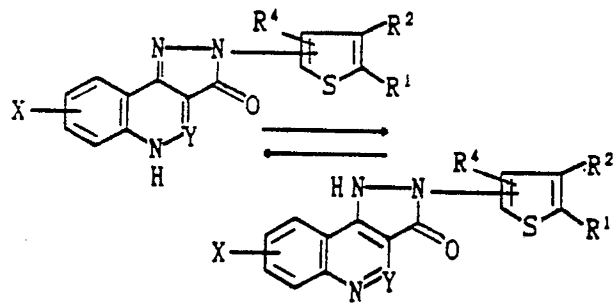

wherein $R^1$, $R^2$, $R^4$, X and Y have the same meanings as used in the definition of formula (I).

The objective compounds of formula (I) and their salts have a high affinity to benzodiazepine-receptors. Thus, they can be used for preparing pharmaceutical compositions having psychotropic activity, such as activators of mentation, minor tranquilizers, anticonvulsants, or agents for treating benzodiazepine intoxication.

The pharmaceutical composition can be formulated as tablets, capsules, pills, granules, injection preparations, suppositories, and syrups in a conventional manner. Lactose, Saccharose, wheat starch, potato starch, magnesium stearate, gelatin, methyl cellulose, agar and water may be used as pharmaceutically acceptable excipients. If required, stabilizers, emulsifiers, buffers and other additives can be added.

The pharmaceutical compositions can be administered orally or parentally to human beings or other mammals. A dose or doses of 0.1—500 mg of active compound per day is suitable for oral administration.

The present invention will be explained in more detail by the following Examples. Reference Examples, and Preparations.

The abbreviations used therein have the following meanings.

Me: methyl; Et: ethyl; Bu: butyl; (d): decomposition point.

Example 1

(1) 2-[2-(3-Ethoxycarbonyl-5-methylthienyl)]-2,5-dihydro-3H-pyrazolo[4,3-c]quinolin-3-one Ia-1

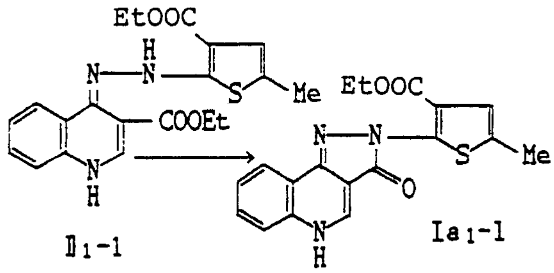

$II_1$-1 $Ia_1$-1

To a suspension of 1.04 g of ethyl 4-[2-(3-ethoxycarbonyl-5-methylthienyl)hydorazono]-1,4-dihydroquinoline-3-carboxylate $II_1$-1 in 15 ml of ethanol is added 4 ml of 1N sodium hydroxide under nitrogen gas at room temperature. The mixture is stirred for 30 minutes, acidified with acetic acid, and dried under reduced pressure. The residue is mixed with water, filtered, and washed with water and ethanol. The resulting solid is crystallized from chloroform-ethyl acetate to givde 1.17 g of a yellowish crystalline substance $1a_1$-1. This is recrystallized from chloroform-ethyl acetate to give yellowish crystals, melting at 248~250°C (d).

(2) 2-[2-(3-Carboxy-5-methylthienyl)]-2,5-dihydro-3H-pyrazolo[4,3-c]quinolin-3-one $1b_1$ 1

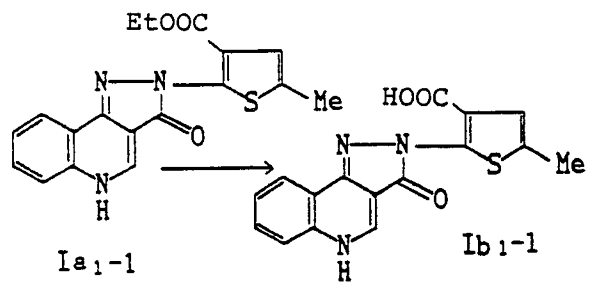

$Ia_1$-1 $Ib_1$-1

To a suspension of 707 mg of 2-[2-(3-ethoxycarbonyl-5-methylthienyl)]-2,5-dihydro-3H-pyrazolo[4,3-c]quinolin-3-one $Ia_1$ -1 in 10 ml of methanol is added 10 ml of 1N sodium hydroxide. The mixture is stirred at 60°C for 1 hour, cooled, neutralized with 10 ml of 1N hydrochloric acid, and acidified with acetic acid. The precipitating crystalline material is collected by filtration, washed with water, and dried to give 637 mg of yellowish crystals $Ib_1$-1. m.p.: 300—303°C (d).

$Ib_1$-1 can be directly produced from $IIb_1$-1 by using the same treatment as in this Example, but without isolation of the intermediate compound $Ia_1$-1.

Examples 2—13

(1)

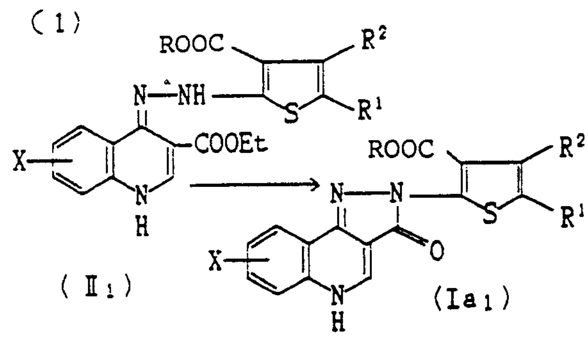

($II_1$) ($Ia_1$)

To a suspension of an ethyl 4-[2-(3-alkoxycarbonylthienyl)-hydrazono]-1,4-dihydroquinoline-3-carboxylate ($II_1$) in ethanol is added aqueous 1N sodium hydroxide under nitrogen gas at room temperature. The mixture is stirred for a period of several tens of minutes to several hours, acidified with acetic acid, and dried under reduced pressure. The residue is washed with water and ethanol in succession;

the resulting solid is crystallized from chloroform-ethyl acetate to give a yellow crystalline compound ($Ia_1$).

(2)

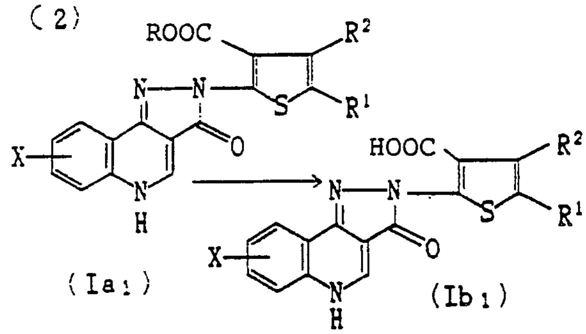

($Ia_1$) ($Ib_1$)

To a supension of a 2-[2-(3-alkoxycarbonylthienyl]-2,5-dihydro-3H-pyrazolo[4,3-c]quinolin-3-one ($Ia_1$) in methanol is added aqueous 1N sodium hydroxide under nitrogen gas. The mixture is stirred under heating for a period of several tens of minutes to several hours. After cooling, the mixture is neutralized with 1N hydrochloric acid and acidified with acetic acid. The precipitating crystals are collected by filtration, washed with water, and dried to give a 2-[2-(3-carboxythienyl)]-2,5-dihydro-3H-pyrzolo[4,3-c]quinolin-3-one ($Ib_1$).

The reaction conditions to prepare Compound ($Ia_1$) from corresponding Compound ($II_1$), as well as data regarding the yield (g), (%), structural formula, recrystallization solvent, and melting point of compound ($Ia_1$) are summarized in Table 1.

The reaction conditions to prepare Compound ($Ib_1$) from corresponding Compound ($Ia_1$), as well as data regarding the yield (g), (%), structural formula, appearance, and decomposition point of compound ($Ib_1$) are shown in Table 2.

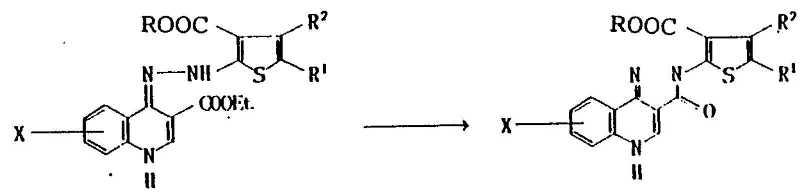

($II_1$) → ($Ia_1$)

Table 1

| Ex. No. | Amount of Compound ($II_1$) (g) | ethanol (ml) | 1NNaOH (ml) | reaction time (hrs) | Compound ($Ia_1$) yield (g) | yield (%) | Compd. No. | $R^1$ | $R^2$ | R | X | crystal water | recrystallization solvent | m.p. (°C) |
|---|---|---|---|---|---|---|---|---|---|---|---|---|---|---|
| 2 | 0.82 | 10 | 2.2 | 0.5 | 0.66 | 90 | $Ia_1$-2 | Et | H | Et | H | 1/4 $H_2O$ | $CHCl_3$-EtOAc | 175-178 |
| 3 | 0.92 | 8 | 2.5 | 1 | 0.52 | 66 | $Ia_1$-3 | Me | Me | Et | H | – | $CHCl_3$, MeOH | 252-253 |
| 4 | 0.63 | 7 | 1.6 | 0.8 | 0.475 | 88 | $Ia_1$-4 | $-(CH_2)_4-$ | | Et | H | – | $CHCl_3$,MeOH | 281-284 |
| 5 | 2.14 | 70 | 10 | 1 | 1.80 | 93 | $Ia_1$-5 | n-Bu | H | Et | H | – | n-hexane-EtOH | 210-212 |
| 6 | 1.04 | 20 | 2.9 | 1 | 0.88 | 96 | $Ia_1$-6 | Me | H | Me | 8-Me | $H_2O$ | MeOH | 162-164d |
| 7 | 1.15 | 50 | 3.4 | 1 | 0.93 | 91 | $Ia_1$-7 | Me | H | Me | 8-MeO | – | MeOH | 255-259 |
| 8 | 0.98 | 20 | 2.6 | 1.5 | 0.80 | 92 | $Ia_1$-8 | Me | H | Me | 8-Cl | $H_2O$ | MeOH | 166-169 |
| 9 | 1.05 | 20 | 2.9 | 1 | 0.865 | 93 | $Ia_1$-9 | Me | H | Me | 8-F | 1/5 $H_2O$ | MeOH-benzene | 168-171 |
| 10 | 0.70 | 5 | 1.9 | 1 | 0.55 | 88 | $Ia_1$-10 | Me | H | Me | 7-Me | – | EtOH | 246-248 |
| 11 | 1.67 | 100 | 8.0 | 1 | 1.36 | 93 | $Ia_1$-11 | Me | H | Me | 7-Cl | – | EtOH | 261-263 |
| 12 | 0.472 | 5 | 1.2 | 0.7 | 0.386 | 90 | $Ia_1$-12 | COOEt | Me | Et | H | – | $CHCl_3$, EtOH | 282-285 |
| 13 | 2.36 | 50 | 6.6 | 0.5 | 1.99 | 94 | $Ia_1$-13 | H | COOEt | Et | H | 1/3 $H_2O$ | MeOH | 255-258 |

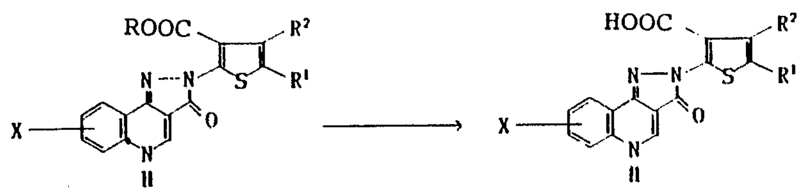

(Ia₁) (Ib₁)

Table 2

| Ex. No. | Amount of Compound (Ia₁) ( g ) | methanol ( ml ) | 1NNaOH ( ml ) | reaction tempera-ture ( °C ) | reaction time (hrs) | Compound ( Ib₁ ) yield ( g ) | yield ( % ) | Compd. No. | $R^1$ | $R^2$ | X | crystal water | appearance | dec. point ( °C ) |
|---|---|---|---|---|---|---|---|---|---|---|---|---|---|---|
| 2 | 0.558 | 10 | 10 | 40 | 1.5 | 0.481 | 92 | Ib₁-2 | Et | H | H | 1/2 $H_2O$ | yellow | 280 283 |
| 3 | 0.500 | 5 | 10 | reflux | 2 | 0.35 | 76 | Ib₁-3 | Me | Me | H | 1/2 $H_2O$ | yellow | 289 291 |
| 4 | 0.48 | 5 | 8 | reflux | 2 | 0.429 | 96 | Ib₁-4 | $-(CH_2)_4-$ | | H | 1/2 $H_2O$ | yellow | 294 296 |
| 5 | 0.70 | 10 | 5.4 | reflux | 0.5 | 0.630 | 95 | Ib₁-5 | n Bu | H | H | 1/2 $H_2O$ | yellow | 280 281 |
| 6 | 0.82 | 8 | 10 | 60 | 0.5 | 0.716 | 91 | Ib₁-6 | Me | H | 8-Me | 1/5 $H_2O$ | yellow | 293 294 5 |
| 7 | 0.88 | 13 | 7.2 | reflux | 0.5 | 0.710 | 84 | Ib₁-7 | Me | H | 8-MeO | 2/5 $H_2O$ | yellow | 295 2963 |
| 8 | 0.73 | 7 | 10 | 60 | 0.5 | 0.672 | 96 | Ib₁-8 | Me | H | 8-Cl | $H_2O$ | yellow | 312 314 |
| 9 | 0.79 | 8 | 10 | 60 | 0.6 | 0.683 | 86 | Ib₁-9 | Me | H | 8-F | 3/5 $H_2O$ | yellow | 299-301 |
| 10 | 0.436 | 8 | 4 | reflux | 0.5 | 0.375 | 86 | Ib₁-10 | Me | H | 7-Me | 1/2 $H_2O$ | yellowish green | 308-311 |
| 11 | 1.19 | 18 | 9.6 | reflux | 0.5 | 1.10 | 95 | Ib₁-11 | Me | H | 7-Cl | 1/2 $H_2O$ | yellowish green | 316-317 |
| 12 | 0.638 | 15 | 15 | 70 | 1.5 | 0.541 | 98 | Ib₁-12 | COOH | Me | H | 3/5 $H_2O$ | yellowish-green | 248 251 |
| 13 | 0.350 | 5 | 5 | 60 | 1.5 | 0.229 | 75 | Ib₁-13 | Cl | COOH | H | 1/2 $H_2O$ | yellow | > 310 |

*Ib₁-13 is prepared from Ia₁-13' which is produced in Example 58.

Example 14

2-[2-(5-Methylthienyl)]-2,5-dihydro-3H-pyrazolo-[4.3-c]quinolin-3-one $Ic_1$-1

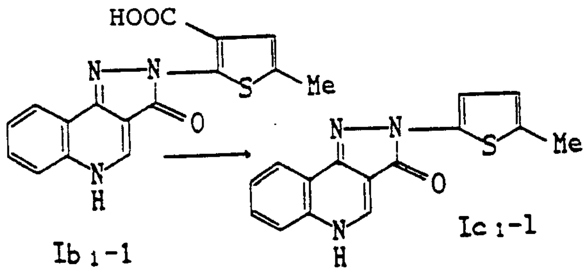

$Ib_1$-1 → $Ic_1$-1

To a suspension of 390 mg of 2-[2-(3-carboxy-5-methylthienyl)]-2,5-dihydro-3H-pyrazolo[4,3-c]quinolin-3-one $Ib_1$-1 in 6 ml of quinoline is added 150 mg of copper powder. The mixture is stirred under nitrogen gas at 200°C for 45 minutes. After cooling, the copper powder is removed by filtration and the filtrate is mixed with 1N sodium hydroxide and shaken with ether. The separated aqueous layer is filtered; and the filtrate is mixed with acetic acid. The precipitating crystals are collected by filtration and recrystallized from ethanol to give 280 mg of $Ic_1$-1 as yellow crystals.
m.p.: 309—311°C (d).

Examples 15—26

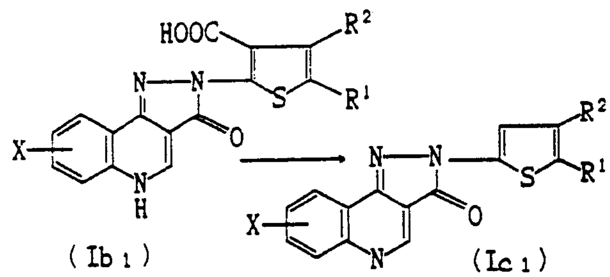

($Ib_1$) → ($Ic_1$)

To a suspension of the compound ($Ib_1$) in quinoline is added copper powder. The mixture is stirred at a temperature of heating under nitrogen gas for a period of several tens of minutes to several hours. After cooling, copper powder is removed by filtration, and the filtrate is mixed with 1N sodium hydroxide and shaken with ether. The separated aqueous layer is filtered; and the filtrate mixed with acetic acid. The precipitating crystalline material is by filtration and crystallized from an appropriate solvent to give the compound ($Ic_1$). The reaction conditions to prepare compound ($Ic_1$) from compound ($Ib_1$); as well as data regarding the yield (g), (%), structural formula, recrystallization solvent and decomposition point of compound ($Ic_1$) are shown in Table 3.

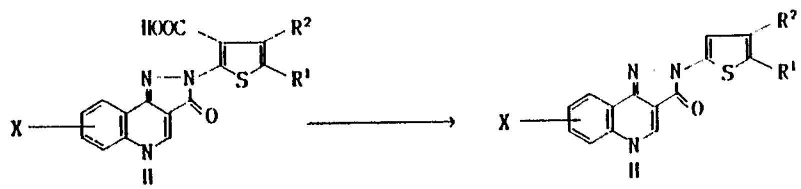

(Ib1) (Ic1)

Table 3

| Ex. No. | Amount of Compound (Ib1) (g) | quinoline (ml) | copper powder (g) | reaction temperature (°C) | reaction time (min) | Compound (Ic1) yield (mg) | yield (%) | Compd. No. | $R^1$ | $R^2$ | X | crystal water | recrystallization solvent | dec. point (°C) |
|---|---|---|---|---|---|---|---|---|---|---|---|---|---|---|
| 15 | 0.348 | 6 | 0.15 | 195 | 90 | 273 | 91 | $Ic_1$-2 | Et | H | H | 1/4 $H_2O$ | EtOH | 277-280 |
| 16 | 0.339 | 5 | 0.12 | 195 | 20 | 182 | 63 | $Ic_1$-3 | Me | Me | H | $H_2O$ | EtOH | 323-328 |
| 17 | 0.42 | 8 | 0.14 | 200 | 40 | 190 | 52 | $Ic_1$-4 | $-(CH_2)_4-$ | | H | 1/2 $H_2O$ | EtOH | 343-346 |
| 18 | 0.335 | 4 | 0.15 | 195 | 35 | 206 | 70 | $Ic_1$-5 | n-Bu | H | H | – | EtOH | 266-270 |
| 19 | 0.66 | 10 | 0.20 | 200 | 40 | 552 | 96 | $Ic_1$-6 | Me | H | 8-Me | – | $CHCl_3$-MeOH | 330-333 |
| 20 | 0.635 | 7 | 0.22 | 195 | 60 | 340 | 62 | $Ic_1$-7 | Me | H | 8-MeO | – | EtOH | 301-302 |
| 21 | 0.62 | 10.5 | 0.18 | 200 | 50 | 467 | 86 | $Ic_1$-8 | Me | H | 8-Cl | – | EtOH | 328-333 |
| 22 | 0.64 | 9.6 | 0.19 | 200 | 50 | 480 | 86 | $Ic_1$-9 | Me | H | 8-F | – | $CHCl_3$, MeOH | 320-325 |
| 23 | 0.70 | 7 | 0.24 | 195 | 60 | 372 | 63 | $Ic_1$-10 | Me | H | 7-Me | – | EtOH | 322-325 |
| 24 | 0.60 | 6 | 0.20 | 195 | 60 | 331 | 63 | $Ic_1$-11 | Me | H | 7-Cl | – | EtOH | 320-325 |
| 25 | 0.517 | 6 | 0.20 | 195 | 25 | 319 | 81 | $Ic_1$-12 | H | Me | H | – | EtOH | 310-312 |
| 26 | 0.200 | 4 | 0.20 | 200 | 20 | 114 | 80 | $Ic_1$-13 | Cl | H | H | $H_2O$ | $CHCl_3$, MeOH | 309-312 |

Example 27

(1) 2-[3-(2-Methoxycarbonylthienyl)]-2,5-dihydro-3H-pyrazolo-[4,3-c]quinolin-3-one $Ia_2$-1

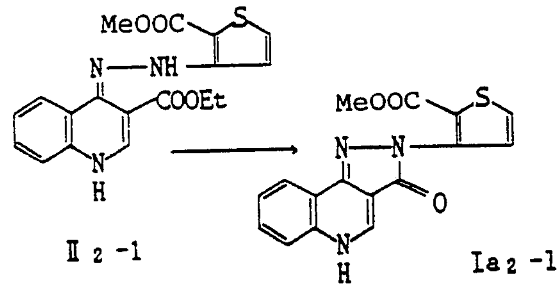

II 2 -1 Ia 2 -1

To a suspension of 1.04 g of ethyl 4-[3-(2-methoxycarbonylthienyl)hydrazono]-1,4-dihydroquinoline-3-carboxylate in 14 ml of ethanol (1.04 g) is added 3.4 ml of 1N sodium hydroxide under nitrogen gas at room temperature. The mixture is stirred for 10 minutes, acidified with acetic acid, and dried under reduced pressure. The residue is mixed with water, filtered, washed with water, and recrystallized from ethanol to give 0.93 g (yield: 96%) of $Ia_2$-1 as yellow crystals.
m.p.: 240—241°C.

(2) 2-[3-(2-Carboxythienyl)]-2,5-dihydro-3H-pyrazolo[4.3-c]quinolin-3-one $Ib_2$-1

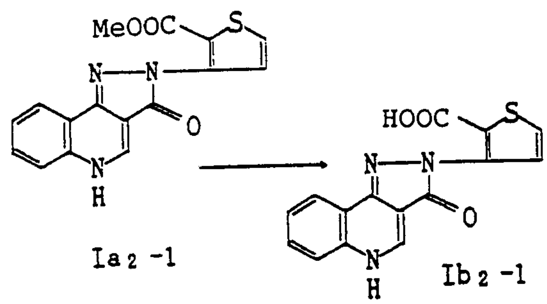

Ia 2 -1 Ib 2 -1

To a suspension of 515 mg of 2-[3-(2-methoxycarbonylthienyl)]-2,5-dihydro-3H-pyrazolo[4,3-c]quinolin-3-one $Ia_2$-1 in 7.5 ml of methanol is added 7.5 ml of 1N sodium hydroxide. The mixture is stirred at 40°C for 1 hour and cooled. The resulting mixture is mixed with acetic acid; and the precipitating crystals are collected by filtration, washed with water, and dried to give 452 mg of $Ib_2$-1 as yellowish green crystals.
m.p.: 263—265 (d).

Compound $Ib_2$-1 can directly be prepared by reacting compound $II_2$-1 in the above manner.

Examples 28—37

(1)

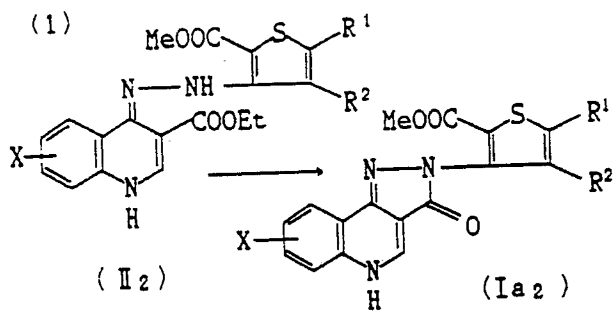

( II 2 ) (Ia 2 )

To a suspension of an ethyl 4-[3-(2-methoxycarbonylthienyl)-hydrazono]-1,4-dihydroquinoline-3-carboxylate ($II_2$) in ethanol is added 1N sodium hydroxide under nitrogen gas at room temperature. The mixture is stirred for a period from several tens of minutes to several hours, acidified with acetic acid, and dried under reduced pressure. The residue is mixed with water, filtered, washed with water, and

recrystallized from an appropriate solvent to give a yellow crystalline compound ($Ia_2$).

(2)

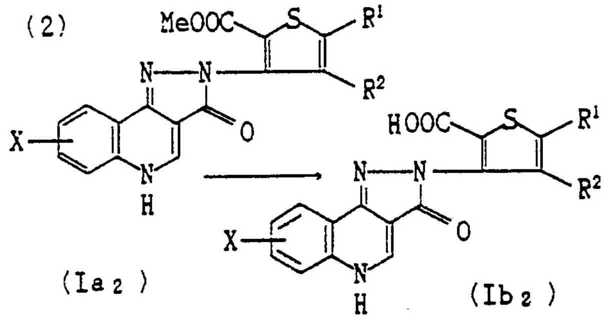

($Ia_2$) ($Ib_2$)

To a suspension of 2-[3-(2-methoxycarbonylthienyl)]-2,5-dihydro-3H-pyrazolo[4,3-c]quinolin-3-one ($Ia_2$) in methanol is added 1N sodium hydroxide under nitrogen gas. The mixture is stirred for a period of about several 10 minutes to several hours with heating. After cooling, the reaction mixture is mixed with acetic acid; and the precipitating crystals are collected by filtration, washed with water, and dried to give a 2-[3-(2-carboxythienyl)]-2,5-dihydro-3H-pyrazolo[4,3-c]quinolin-3-one ($Ib_2$) as yellow crystals.

The reaction conditions to prepare compound ($Ia_2$) from the corresponding compound ($II_2$), as well as data ragarding the yield (g), (%), structural formula, recrystallization solvent, and melting point of compound ($Ia_2$) are shown in Table 4; the reaction conditions to prepare compound ($Ib_2$) from the corresponding compound ($Ia_2$), as well as data regarding the yield, structural formula, and decomposition point of compound ($Ib_2$) are shown in Table 5.

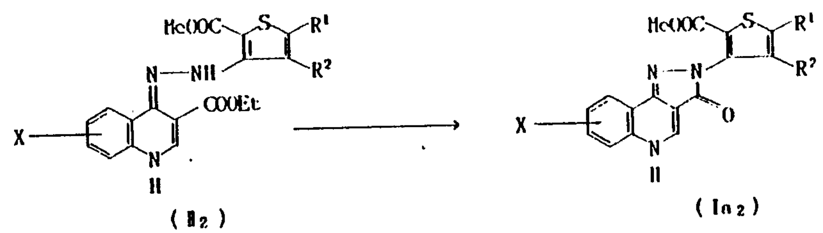

( $II_2$ ) ( $Ia_2$ )

Table 4

| Ex. No. | Amount of Compound ( $II_2$ ) ( g ) | ethanol ( ml ) | 1NNaOH ( ml ) | reaction time ( min ) | Compound ( $Ia_2$ ) yield ( g ) | yield ( % ) | Compd. No. | $R^1$ | $R^2$ | X | crystal water | recrystalli zation solvent | m.p. ( °C ) |
|---|---|---|---|---|---|---|---|---|---|---|---|---|---|
| 28 | 13.4 | 200 | 38 | 60 | 11.50 | 97 | $Ia_2$-2 | Me | H | H | $H_2O$ | EtOH | 260 261 |
| 29 | 0.74 | 8 | 2.2 | 50 | 0.573 | 88 | $Ia_2$-3 | H | Me | H | – | EtOH | 265-267d |
| 30 | 0.95 | 19 | 2.6 | 80 | 0.66 | 79 | $Ia_2$-4 | Me | H | 8-Me | – | MeOH | 179 181d |
| 31 | 1.20 | 70 | 3.8 | 60 | 1.04 | 98 | $Ia_2$-5 | Me | H | 8-MeO | – | MeOH | 275 277 |
| 32 | 0.37 | 15 | 1.0 | 90 | 0.285 | 87 | $Ia_2$-6 | Me | H | 8-Cl | 1/2 $H_2O$ | EtOH | 195 199 |
| 33 | 1.02 | 25 | 2.9 | 90 | 0.865 | 96 | $Ia_2$-7 | Me | H | 8-F | $H_2O$ | MeOH | 189 191d |
| 34 | 0.945 | 50 | 4.7 | 120 | 0.575 | 69 | $Ia_2$-8 | Me | H | 7-Me | 3/4 $H_2O$ | MeOH | 153 156 |
| 35 | 1.45 | 60 | 6.9 | 90 | 1.04 | 81 | $Ia_2$-9 | Me | H | 7-Cl | – | EtOH | 167-169 |
| 36 | 0.66 | 5 | 1.8 | 60 | 0.55 | 94 | $Ia_2$-10 | Et | H | H | - | EtOH | 150 155 |
| 37 | 3.118 | 50 | 8.3 | 120 | 2.28 | 82 | $Ia_2$-11 | Me | H | 7 MeO | - | EtOH | 223 230 |

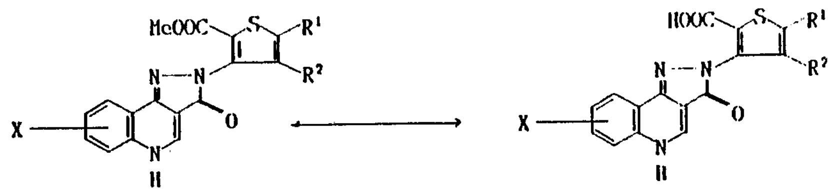

(Ia2)  (Ib2)

Table 5

| Ex. No. | Amount of Compound ($Ia_2$) (g) | methanol (ml) | 1NNaOH (ml) | reaction temperature (°C) | reaction time (min) | Compound (Ib2) yield (g) | yield (%) | Compd. No. | $R^1$ | $R^2$ | X | crystal water | dec point (°C) |
|---|---|---|---|---|---|---|---|---|---|---|---|---|---|
| 28 | 10.90 | 200 | 96 | reflux | 60 | 9.70 | 93 | $Ib_2$-2 | Me | H | H | 1/3 $H_2O$ | 260-261 |
| 29 | 0.53 | 5 | 10 | 60 | 60 | 0.465 | 91 | $Ib_2$-3 | H | Me | H | 1/4 $H_2O$ | 297-299 |
| 30 | 0.62 | 6 | 10 | 60 | 50 | 0.495 | 83 | $Ib_2$-4 | Me | H | 8-Me | 2/5 $H_2O$ | 321-324 |
| 31 | 0.955 | 15 | 7.8 | reflux | 40 | 0.82 | 90 | $Ib_2$-5 | Me | H | 8-MeO | -- | 307-312 |
| 32 | 0.67 | 6 | 10 | 60 | 40 | 0.592 | 92 | $Ib_2$-6 | Me | H | 8-Cl | 5/4 $H_2O$ | 344-347 |
| 33 | 0.15 | 1.5 | 2 | 50 | 30 | 0.125 | 87 | $Ib_2$-7 | Me | H | 8-F | $H_2O$ | 324-328 |
| 34 | 0.513 | 8 | 5 | reflux | 30 | 0.41 | 85 | $Ib_2$-8 | Me | H | 7-Me | 1/2 $H_2O$ | 301-304 |
| 35 | 0.917 | 15 | 7.4 | reflux | 30 | 0.833 | 94 | $Ib_2$-9 | Me | H | 7-Cl | - | 337-340 |
| 36 | 0.44 | 8 | 3.7 | reflux | 60 | 0.42 | 98 | $Ib_2$-10 | Et | H | H | $H_2O$ | > 300 |
| 37 | 4.076 | 66 | 33 | reflux | 80 | 3.72 | 95 | $Ib_2$-11 | Me | H | 7-MeO | 1/3 $H_2O$ | > 300 |

Example 38

2-(3-Thienyl)-2,5-dihydro-3H-pyrazolo[4,3-c]quinolin-3-one $IC_2$-1

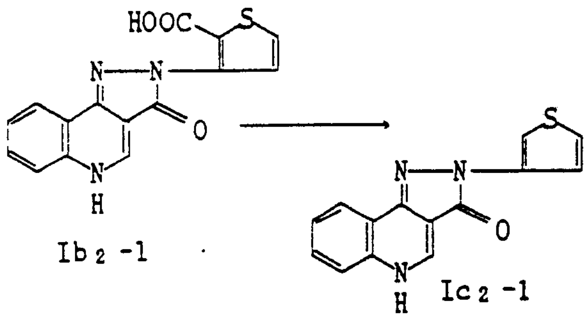

$Ib_2$-1 $Ic_2$-1

To a suspension of 400 mg of 2-[3-(2-carboxythienyl)]-2,5-dihydro-3H-pyrazolo[4,3-c]quinolin-3-one $Ib_2$-1 in 4 ml of quinoline is added 200 mg of copper powder. The mixture is stirred under nitrogen gas as 195°C for 10 minutes; and the copper powder is removed by filtration. The filtrate is mixed with 1N sodium hydroxide, and extracted with ether to remove quinoline. The separated aqueous layer is filtered and the filtrate is mixed with acetic acid. The precipitating crystals are collected by filtration, washed with ester, and recrystallized from ethanol to give 321 mg of $Ic_2$-1 as yellow crystals.
m.p.: 323—325°C (d).

Examples 39—48

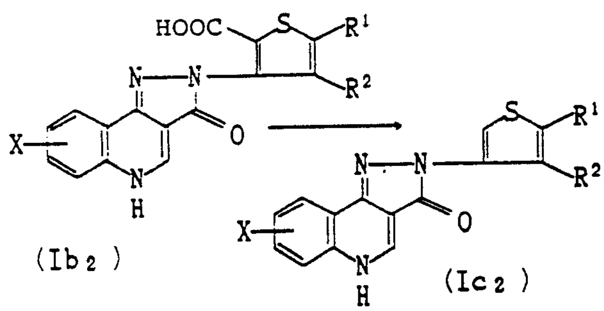

($Ib_2$) ($Ic_2$)

To a suspension of compound ($Ib_2$) in quinoline is added copper powder. The mixture is stirred under nitrogen gas with heating for a period of about several 10 minutes to about several hours. After cooling, the copper powder is removed by filtration. The filtrate is mixed with 1N sodium hydroxide and extracted with ether. The separated aqueous layer is filtered, and the filtrate is mixed with acetic acid. The precipitating crystals are collected by filtration, washed with water, and recrystallized from an appropriate solvent to give compound ($Ic_2$) as yellow crystals.

The reaction conditions to prepare compound ($Ic_2$) from the corresponding compound ($Ib_2$), as well as data regarding the yield, structural formula, recrystallization solvent, and decomposition point of compound ($Ic_2$) are shown in Table 6.

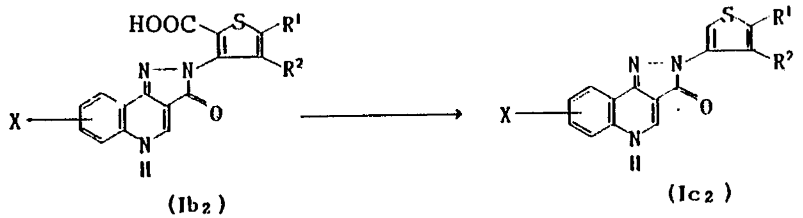

(Ib₂) (Ic₂)

Table 6

| Ex. No. | Amount of Compound ($Ib_2$) (g) | quinoline (ml) | copper powder (g) | reaction temperature (°C) | reaction time (min) | Compound ($Ic_2$) yield (g) | yield (%) | Compd. No. | $R^1$ | $R^2$ | X | crystal water | recrystallization solvent | dec. point (°C) |
|---|---|---|---|---|---|---|---|---|---|---|---|---|---|---|
| 39 | 5.00 | 50 | 1.50 | 190 | 60 | 3.60 | 85 | $Ic_2$-2 | Me | H | H | 1/4 $H_2O$ | EtOH | 298-300 |
| 40 | 0.41 | 8 | 0.12 | 200 | 30 | 0.33 | 93 | $Ic_2$-3 | H | Me | H | 1/2 $H_2O$ | EtOH | 313-315 |
| 41 | 0.45 | 9 | 0.135 | 195 | 40 | 0.32 | 82 | $Ic_2$-4 | Me | H | 8-Me | – | MeOH | 330-332 |
| 42 | 0.737 | 7.4 | 0.24 | 195 | 70 | 0.515 | 80 | $Ic_2$-5 | Me | H | 8-MeO | $H_2O$ | EtOH | 322-324 |
| 43 | 0.43 | 8 | 0.13 | 195 | 40 | 0.303 | 81 | $Ic_2$-6 | Me | H | 8-Cl | – | EtOH-$CHCl_3$ | 349-352 |
| 44 | 0.54 | 8 | 0.16 | 200 | 40 | 0.405 | 86 | $Ic_2$-7 | Me | H | 8-F | – | MeOH | 326-329 |
| 45 | 0.392 | 5 | 0.13 | 195 | 60 | 0.23 | 68 | $Ic_2$-8 | Me | H | 7-Me | 3/4 $H_2O$ | EtOH | 311-314 |
| 46 | 0.70 | 10 | 0.24 | 195 | 60 | 0.402 | 65 | $Ic_2$-9 | Me | H | 7-Cl | – | – | 323-327 |
| 47 | 0.40 | 3 | 0.13 | 190 | 120 | 0.28 | 80 | $Ic_2$-10 | Et | H | H | 1/3 $H_2O$ | EtOH | 266-268 |
| 48 | 3.67 | 33 | 1.13 | 195 | 110 | 2.80 | 86 | $Ic_2$-11 | Me | H | 7-MeO | 1/4 $H_2O$ | EtOH | 317-321 |

Example 49

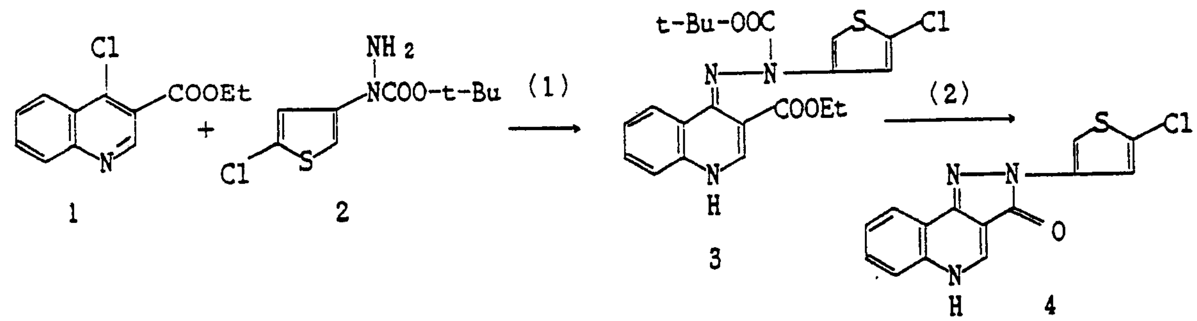

2-[3-(5-Chlorothienyl)]-2,5-dihydro-3H-pyrazolo[4,3-c]quinolin-3-one 4

(1) To a solution of 400 mg of ethyl 4-chloroquinoline-3-carboxylate 1 in 10 ml of ethanol is added 380 mg of $N^1$-tert-butoxycarbonyl-$N^1$-[3-(5-chlorothienyl)]hydrazine 2. The mixture is stirred at 40°C for 1 hour and concentrated. The resulting residue is mixed with chloroform, washed with aqueous sodium bicarbonate and water, dried with anhydrous magnesium sulfate, and concentrated. The residue is purified by silica-gel column chromatography (benzene:ethyl acetate = 30:1) to give 670 mg (yield: 93%) of ethyl 4-[$N^2$-3-(5-chlorothienyl)-$N^2$-t-butoxycarbonyl]hydrazinoquinoline-3-carboxylate 3.

m.p.: 134—135°C.

(2) To a solution of 600 mg of crystalline compound 3 in 15 ml of dichloromethane is added 10 ml of trifluoroacetic acid. The mixture is stirred at 50°C for 20 minutes and concentrated. The resulting residue is dissolved in 30 ml of ethanol and mixed with 8 ml of 1N sodium hydroxide under ice-cooling. The reaction mixture is stirred at room temperature for 1.5 hours and concentrated. The residue is mixed with 10 ml of water and extracted with ether to remove the fat-soluble portion. The aqueous layer is filtered; and the filtrate is mixed with acetic acid. The precipitating solid is collected by filtration and recrystallized from ethanol to give 210 mg of 2-[3-(5-chlorothienyl)]-2,5-dihydro-3H-pyrazolo[4,3-c]quinolin-3-one 4.

Yield: 52%.

m.p.: 287—289°C.

NMR (DMSO-$d_6$): 7.45—7.83 (5H, m); 8.17—8.27 (1H, m), 8.75 (1H, s).

| | | | |
|---|---|---|---|
| Anal. Calcd. (%) (for $C_{14}H_8N_3OSCl$): | C, 55.73; | H, 2.68; | N, 13.93 |
| Found (%): | C, 55.56; | H, 2.99; | N, 13.56 |

The reagent $N_1$-tert-butoxycarbonyl-$N^1$-[3-(5-chlorothienyl)]hydrazine 2 is prepared by subjecting 3-tert-butoxycarbonylamino-5-chlorothiophene (m.p.: 86—87°C) to amination according to Synthesis. 487, *1977*; and the latter is prepared from 5-chlorothiophene-3-carboxylic acid in the manner described in Synthesis, 255, *1977*.

Example 50

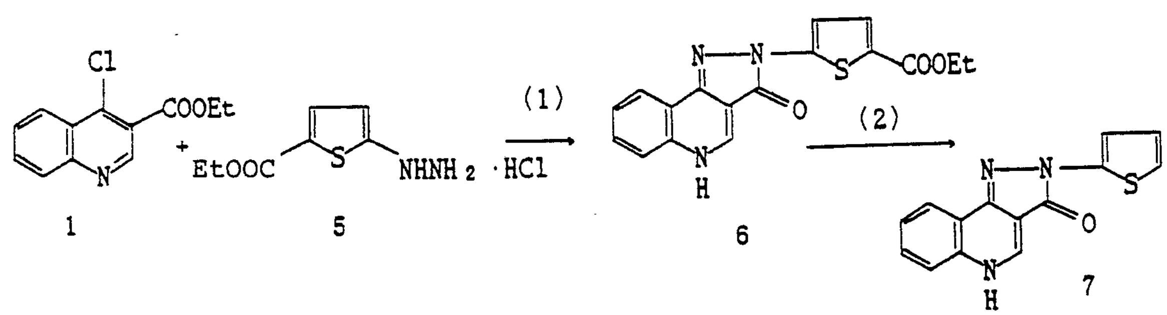

(1) 2-[2-(5-Ethoxycarbonylthienyl)]-2,5-dihydro-3H-pyrazolo[4,3-c]quinolin-3-one 6

To a solution of 942 mg of ethyl 4-chloroquinoline-3-carboxylate 1 in 10 ml of ethanol is added 981 mg of ethyl 5-hydrazinothiophene-2-carboxylate 5 [Can. J. Chem., *44*, 2881 (1966), m.p.: 160—170°C]. The mixture is stirred at 50—55°C for 30 minutes and concentrated. The resulting mixture is mixed with sodium bicarbonate, extracted with chloroform, washed with water dried, and evaporated. The residue is purified by silica-gel column chromatography to give 645 mg (48%) of the title compound 6 as crystals, m.p.: higher than 300°C.

NMR (DMSO-$d_6$) δ: 1.31 (3H, t), 4.29 (2H, q), 7.38 (1H, d), 7.50—7.89 (4H, m), 8.19—8.30 (1H, m), 8.88 (1H, s)

(2) 2-(2-Thienyl)-2,5-dihydro-3H-pyrazolo[4,3-c]quinolin-3-one 7

To a suspension of 340 mg of compound 6 in 5 ml of methanol is added 5 ml of 1N sodium hydroxide. The mixture is stirred at 50—55°C for 30 minutes. After cooling, the mixture is mixed with 4 ml of 1N hydrochloric acid, and 0.5 ml of acetic acid. The precipitating crystals are collected by filtration washed with water, and dried. The mixture of 280 mg of 2-[2-(5-carboxythienyl)]-2,5-dihydro-3H-pyrazolo[4,3-c]quinolin-3-one. 3 ml of quinoline, and 140 mg of copper powder is heated at 190°C under nitrogen gas for 20 minutes and cooled. After the removal of copper powder, the mixture is shaken with ether and 1N sodium hydroxide. The aqueous layer is separated and acetic acid is added thereto. The precipitating crystals are collected by filtration to give 210 mg (74%) of crystalline compound 7 as monohydrate.

m.p.: higher than 300°C

NMR (DMSO-$d_6$) δ: 6.92—7.14 (2H, m), 7.37 (1H, dd), 7.50—7.75 (3H, m), 8.16—8.27 (1H, m), 8.77 (1H, s)

Example 51

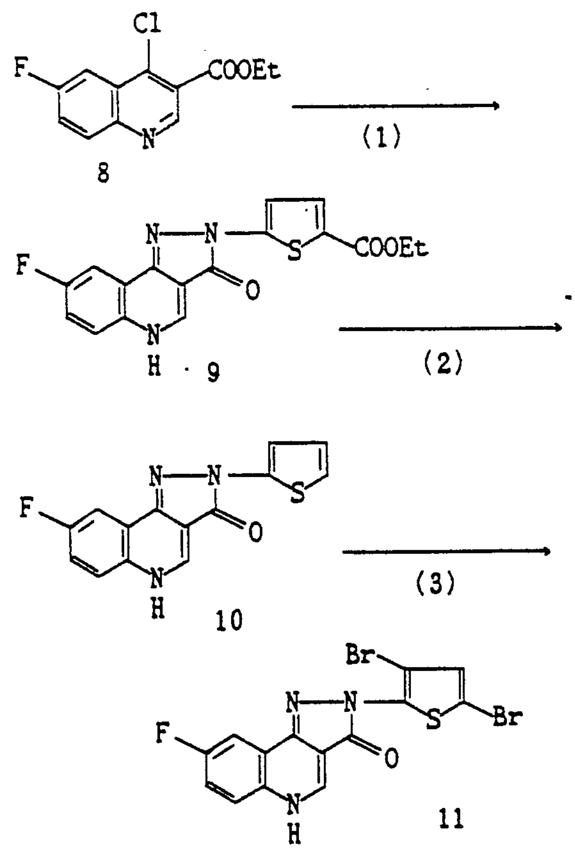

8
9
10
11

(1) 2-[2-(5-Ethoxycarbonylthienyl)]-8-fluoro-2,5-dihydro-3H-pyrazolo[4,3-c]quinolin-3-one 9

A mixture of 1.27 g of ethyl 4-chloro-6-fluoroquinoline-3-carboxylate 8 and 1.11 g of ethyl 5-hydrazinothiophene-2-carboxylate 5 is stirred at 40°C in 50 ml of ethanol for 1 hour. The mixture is treated in the same manner as in Example 50 to give 805 mg (45%) of the title compound 9 as crystals.

m.p.: higher than 300°C

NMR (DMSO-$d_6$) δ: 1.31 (3H, t), 4.28 (2H, q), 7.37 (1H, d), 7.48—7.96 (4H, m), 8.88 (1H, s)

(2) 2-(2-Thienyl)-8-fluoro-2,5-dihydro-3H-pyrazolo[4,3-c]quinolin-3-one 10

A solution of 1.10 g of compound 9 in 22 ml of methanol and 11 ml of 1N sodium hydroxide is heated at 60°C for 1 hour, neutralized with hydrochloric acid, and acidified with acetic acid. The resulting crystals are collected by filtration to give a carboxylic acid. A mixture of the carboxylic acid and 270 mg of copper powder in 14 ml of quinoline is heated at 200°C for 50 minutes. After removal of copper powder, the mixture is shaken with ether and 1N sodium hydroxide. The aqueous layer is separated and mixed with acetic acid. The precipitating crystals are collected by filtration and purified by silica-gel column chromatography to give 370 mg (48%) of compound 10.

m.p.: higher than 300°C

NMR (DMSO-$d_6$) δ: 6.92—7.16 (2H, m), 7.38 (1H, dd), 7.50—7.97 (3H, m), 8.82 (1H, s)

(3) 2-[2-(3,5-Dibromothienyl)]-8-fluoro-2,5-dihydro-3H-pyrazolo[4.3-c]quinoline-3-one 11

A mixture of 330 mg of compound 10 and 495 mg of N-bromosuccinimide in carbon tetrachloride is refluxed for 3.5 hours. The reaction mixture is extracted with aqueous sodium hydroxide; and the aqueous layer is neutralized with hydrochloric acid and acidified with acetic acid. The precipitating crystals are collected by filtration and purified by silica-gel column chromatography to give 315 mg (62%) of compound 11 as crystals.

m.p.: 277—281°C (d)

NMR (DMSO-$d_6$) δ: 7.34 (1H, s), 7.53—7.89 (3H, m), 8.77 (1H, s)

Example 52

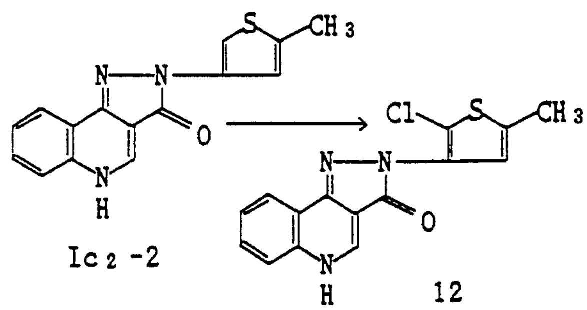

Ic2 -2

12

2-[3-(2-Chloro-5-methylthienyl)]-2,5-dihydro-3H-pyrazolo[4,3-c]quinolin-3-one 12

To a suspension of 110 mg of 2-[3-(5-methylthienyl)]-2,5-dihydro-3H-pyrazolo[4,3-c]quinoline-3-one $Ic_2$—2 in 1 ml of chloroform is added 0.42 ml of a solution of (1.3 M) chlorine in carbon tetrachloride. The mixture is stirred at room temperature for 2 hours. The precipitating crystals are collected by filtration. Dissolved in 1N sodium hydroxide, and mixed with acetic acid. The precipitating crystals are collected by filtration to give 77 mg (60%) of compound 12 as crystals (3/4 mole hydrate).

m.p.: 156—160°C

NMR (DMSO-$d_6$) δ: 2.44 (3H, s), 6.99—7.03 (1H, m), 7.40—8.18 (4H, m), 8.70 (1H, s)

Example 53

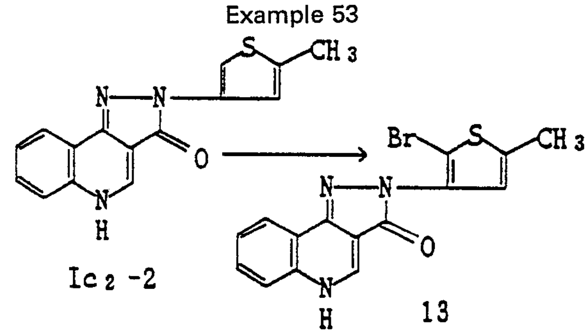

Ic2 -2

13

2-[3-(2-Bromo-5-methylthienyl)]-2,5-dihydro-3H-pyrazolo[4,3-c]quinoline-3-one 13

To a suspension of 196 mg of 2-[3-(5-methylthienyl)]-2,5-dihydro-3H-pyrazolo[4,3-c]quinoline-3-one $Ic_2$-2 in 5 ml of chloroform is dropwise added a solution of 160 mg of bromine in 2 ml of chloroform at room temperature. The mixture is stirred at room temperature for 2 hours. The precipitating crystals are collected by filtration to give 190 mg of compound 13 as a dihydrate.

Yield: 63%

m.p.: 238—243°C (d)

NMR (DMSO-$d_6$) δ: 2.44 (3H, s), 6.93—6.97 (1H, m), 7.40—7.75 (3H, m), 8.05—8.17 (1H, m), 8.68 (1H, s)

Example 54

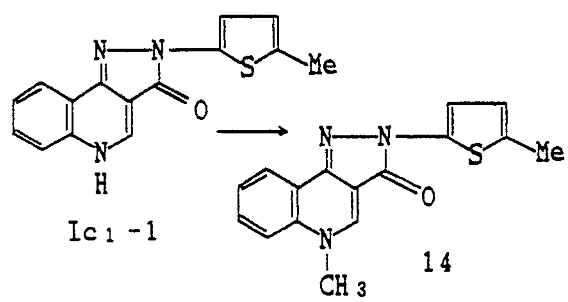

Ic1 -1

14

5-Methyl-2-[2-(5-methylthienyl)]-2,5-dihydro-3H-pyrazolo[4,3-c]quinolin-3-one 14

To a suspension of 400 mg of 2-[2-(5-methylthienyl)]-2,5-dihydro-3H-pyrazolo[4,3-c]quinolin-3-one $IC_1$-1 in 10 ml of anhydrous tetrahydrofuran is added 60 mg of 60% sodium hydride (in mineral oil) under nitrogen gas. The mixture is refluxed for 1.5 hours and mixed with a solution of 283 mg of methyl iodide in 0.5 ml of anhydrous tetrahydrofuran with ice-cooling under stirring. The mixture is stirred at room temperature for 3 hours.

The precipitating crystals are collected by filtration and washed with a mixture of ethanol and ether. The resulting crystals are recrystallized from chloroform-methanol to give 355 mg of compound 14 as crystals.

m.p.: 271—274°C (d)

| | | | |
|---|---|---|---|
| Anal. Calcd. (%) (for $C_{16}H_{13}N_3OS.1/8H_2O$): | C, 64.57; | H, 4.49; | N, 14.12 |
| Found (%): | C, 64.45; | H, 4.65; | N, 14.14 |

NMR (DMSO-$d_6$) δ: 2.40 (3H, d), 4.02 (3H, s), 6.63 (1H, dd), 7.11 (1H, d), 7.50—8.32 (4H, m), 8.87 (1H, s) ppm

Example 55

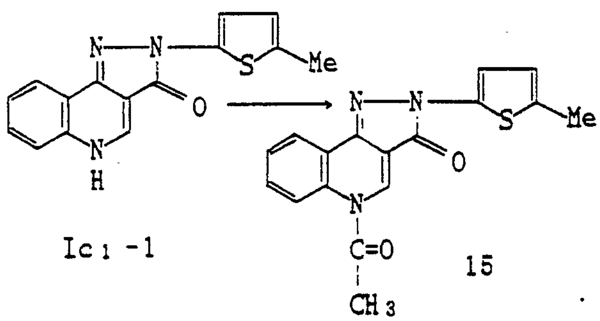

$Ic_1$ -1

15

5-Acetyl-2-[2-(5-methylthienyl)]-2,5-dihydro-3H-pyrazolo[4,3-c]quinolin-3-one 15

To a suspension of 562 mg of 2-[2-(5-methylthienyl)]-2,5-dihydro-3H-pyrazolo[4,3-c]quinolin-3-one $Ic_1$-1 in 10 ml of anhydrous tetrahydrofuran is added 60 mg of 60% sodium hydride (in mineral oil) under nitrogen gas. The mixture is refluxed for 2 hours and mixed with a solution of 196 mg of acetyl chloride in 1 ml of anhydrous tetrahydrofuran under ice-cooling. The mixture is stirred at room temperature for 2 hours; and 0.1 ml of acetic acid is added thereto. The precipitating crystals are collected by filtration, washed with ether-tetrahydrofuran and water, and dried to give 520 mg of the compound 15 as crystals.

m.p.: 306—309°C (d)

| | | | |
|---|---|---|---|
| Anal. Calcd. (%) (for $C_{17}H_{18}N_3O_2S.1/5H_2O$): | C, 62.45; | H, 4.14; | N, 12.85 |
| Found (%): | C, 62.71; | H, 4.42; | N, 12.56 |

NMR (DMSO-$d_6$) δ: 2.43 (3H, s), 2.89 (3H, s), 6.67 (1H, dd), 7.12 (1H, dd), 7.50—8.38 (4H, m), 9.00 (1H, s) ppm

Example 56

A mixture of 281 mg of 2-[2-(5-methylthienyl)]-2,5-dihydro-3H-pyrazolo[4,3-c]quinolin-3-one $Ic_1$-1, 5 ml of trifluoroacetic acid, and 96 mg of methanesulfonic acid is stirred at room temperature for 1.5 hours and dried under reduced pressure. The resulting residue is mixed with ethyl ether and collected by filtration to give 300 mg (yield: 80%) of compound $Ic_1$-1 as methanesulfonate.

m.p.: 230—234°C (d)

| | | | |
|---|---|---|---|
| Anal. Calcd. (%) (for $C_{16}H_{15}N_3O_4S_2.1/2H_2O$): | C, 49.72; | H, 4.17; | N, 10.87 |
| Found (%): | C, 49.72; | H, 4.16; | N, 10.88 |

Example 57

A mixture of 281 mg of 2-[2-(5-methylthienyl)]-2,5-dihydro-3H-pyrazolo[4,3-c]quinolin-3-one $Ic_1$-1 in 2 ml of 0.5 N sodium hydroxide is stirred for 24 hours. The reaction mixture is filtered, and the filtrate is dried under reduced pressure. The resulting residue is washed with ether-ethanol and dried to give 185 mg (yield: 31%) of the sodium salt of compound $Ic_1$-1.

m.p.: 273—277°C (d)

| | | | |
|---|---|---|---|
| Anal. Calcd. (%) (for $C_{15}H_{10}N_3OSNa$): | C, 59.40; | H, 3.32; | N, 13.85 |
| Found (%): | C, 59.68; | H, 3.70; | N, 13.87 |

Example 58

2-{2-[5-chloro-3,4-bis(ethoxycarbonyl)thienyl]}-2,5-dihydro-3H-pyrazolo[4,3-c]quinolin-3-one $Ia_1$-13′

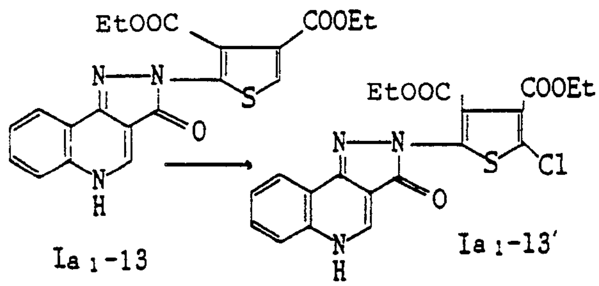

Ia 1-13    Ia 1-13′

To a suspension of 617 mg of 2-{2-[3,4-bis(ethoxycarbonyl)thienyl]}-2,5-dihydro-3H-pyrazolo[4,3-c]-quinoline-3-one $Ia_1$-13 in 20 ml of chloroform is dropwise added 1.4 ml of a solution of 1.35 M chlorine in carbon tetrachloride at 0—5°C. The mixture is stirred at 10—15°C for 40 minutes and filtered; and the filtrate is concentrated. The residue is dissolved in ether and shaken with aqueous sodium hydroxide. The separated aqueous layer is neutralized with acetic acid and the precipitating crystals are collected by filtration to give 289 mg (43%) of compound $Ia_1$-13′ as yellow crystals. $Ia_1$-13′ was utilized to prepare $Ib_1$-13 in Example 13.

m.p.: 152—155°C (d)

Example 59

2-[3-(5-Methyl-2-methoxycarbonylthienyl)]-2,5-dihydro-3H-imidazo-[4,3-c]cinnolin-3-one A

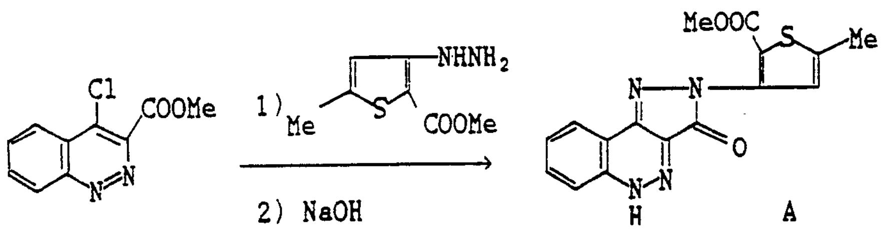


A

A solution of 500 mg of methyl 4-chlorocinnoline-3-carboxylate and 460 mg of methyl 5-methyl-3-hydrazinothiophene-2-carboxylate in 7 ml of ethanol is stirred at room temperature for 30 minutes. The mixture is concentrated and mixed with aqueous ammonia; and the precipitating crystals are filtered off and dried. This compound is stirred in a mixture of methanol (6 ml) -1N sodium hydroxide (1.2 ml) at room temperature for 1 hour and acidified with acetic acid. The resulting crystals are filtered, washed with water, and dried to give 640 mg (86%) of compound A as crystals. This compound is recrystallized from ethanol to give strong red crystals. m.p.: 295—300°C

Example 60

2-[3-(5-Methylthienyl)]-2,5-dihydro-3H-imidazo[4,3-c]cinnolin-3-one B

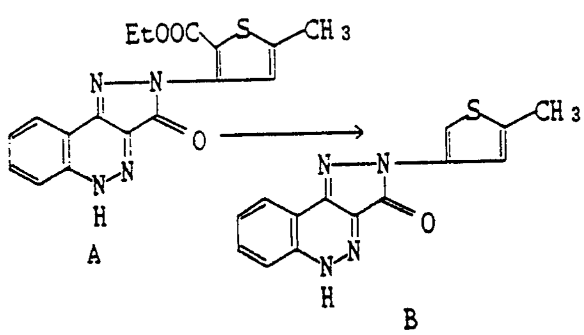

A    B

To a suspension of 330 mg of 2-[3-(5-methyl-2-methoxycarbonylthienyl)]-2,5-dihydro-3H-imidazo[4,3-c]cinnolin-3-one A in 3 ml of ethanol is added 3 ml of 1N sodium hydroxide. The mixture is refluxed for 1.5 hours, cooled and acidified with acetic acid. The precipitating crystals are filtered off and dried. These crystals are suspended in 2.5 ml of quinoline and 95 mg of copper powder and heated at 195°C for 45 minutes. After removal of copper powder, the mixture is shaken with 3 ml of 1 N sodium hydroxide and ether. The separated aqueous layer is filtered by passing through celite. The filtrate is mixed with acetic acid to give 190 mg (74%) of compound B as strong red crystals. m.p.: >310°C

Example 61

2-[2-(5-Methyl-3-methoxycarbonylthienyl)]-2,5-dihydro-3H-pyrazolo[4,3-c]cinnolin-3-one C

A solution of 480 mg of 4-chlorocinnoline-3-carboxylic acid and 441 mg of methyl 5-methyl-2-hydrazinothiophene-2-carboxylate in 6 ml of ethanol is stirred at room temperature for 30 minutes. The reaction mixture is concentrated and mixed with aqueous ammonia; and the precipitating crystals are filtered off and dried. This is added to a mixture of 10 ml of methanol and 1.9 ml of 1N sodium hydroxide. The mixture is stirred at room temperature for 1 hour and acidified with acetic acid. The resulting crystals are filtered off, washed with water, and dried to give 540 mg (73%) of compound C as strong red crystals.

m.p.: 138—140°C

Example 62

2-[2-(5-Methylthienyl)]-2,5-dihydro-3H-pyrazolo[4,3-c]cinnolin-3-one D

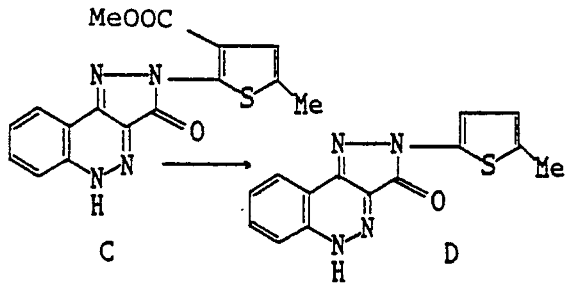

To a suspension of 420 mg of 2-[2-(5-methyl-3-methoxycarbonyl)thienyl)-2,5-dihydro-3H-pyrazolo[4,3-c]cinnolin-3-one C in 8 ml of ethanol is added 4 ml of sodium hydroxide. The mixture is refluxed for 1 hour, cooled, and acidified with acetic acid. The precipitating crystals are filtered off and dried. The crystals are suspended in 3 ml of quinoline and 130 mg of copper powder. The suspension is heated at 195°C for 1 hour. After removal of copper powder, the mixture is shaken with 4 ml of 1N sodium hydroxide. The separated aqueous layer is filtered through celite. The filtrate is mixed with acetic acid and the precipitating crystals are collected by filtration to give 200 mg (72%) of compound D as strong red crystals.

m.p.: >310°C

Examples 63—64

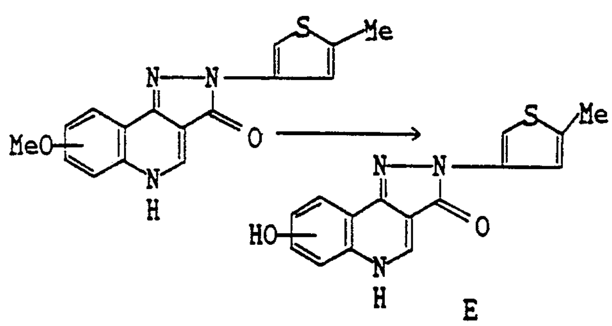

2-[3-(5-Methylthienyl)]-8-hydroxy-2,5-dihydro-3H-pyrazolo[4,3-c]quinolin-3-one $E_1$ (Example 63)

1.066 g of 2-[3-(5-methylthienyl)]-8-methoxy-2,5-dihydro-3H-pyrazolo[4,3-c]quinolin-3-one $Ic_2$-5 is dissolved in 33 ml of quinoline with heating. After cooling, the mixture is mixed with 3 ml of trimethylsilyl iodide and stirred at 190°C for 2 hours. The reaction mixture is mixed with 40 ml of 2N hydrochloric acid, stirred for several hours, basified with Zn sodium hydroxide, and extracted with ether to remove quinoline. The aqueous layer is decolorized with active carbon and mixed with acetic acid. The precipitating crystals are collected by filtration and purified by silica-gel column chromatography. The fractions eluted by chloroform-methanol (50:3) are recrystallized from chloroform-methanol to give 797 mg (89%) of compound $E_1$ as yellow crystals.

m.p.: >300°C

| | | | | |
|---|---|---|---|---|
| Anal. Calcd. (%) (for $C_{15}H_{11}N_3O_2S.CH_3OH$): | C, 58.34; | H, 4.59; | N, 12.75; | S, 9.73 |
| Found (%): | C, 58.14; | H, 4.42; | N, 12.84; | S, 9.19 |

2-[3-(5-Methylthienyl)]-7-hydroxy-2,5-dihydro-3H-pyrazolo[4,3-c]quinolin-3-one $E_2$ (Example 64)

A mixture of 783 mg of 2-[3-(5-methylthienyl)]-7-methoxy-2,5-dihydro-3H-pyrazolo[4,3-c]quinolin-3-one $Ic_2$-11, 25 ml of quinoline, and 2.5 ml of trimethylsilyl iodide is treated in the same manner as in Example 63 to give 389 mg (52%) of compound $E_2$ as yellow crystals.

m.p.: 312—318°C (d)

| | | | | |
|---|---|---|---|---|
| Anal. Calcd. (%) (for $C_{15}H_{11}N_3O_2S.4/5H_3O$): | C, 57.79; | H, 4.07; | N, 13.48; | S, 10.29 |
| Found (%): | C, 57.75; | H, 4.28; | N, 13.41; | S, 9.95 |

Examples 65—67

5-Alkyl-2-[3-(5-methylthienyl)]-2,5-dihydro-3H-pyrazolo[4,3-c]quinolin-3-one F

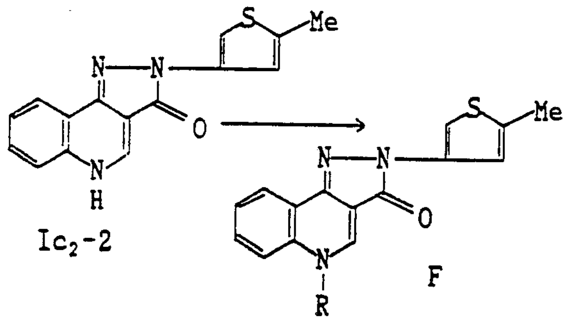

To a suspension of 1562 mg of 2-[3-(5-methylthienyl)]-2,5-dihydro-3H-pyrazolo[4,3-c]quinolin-3-one $Ic_2$-2 in 5 ml of anhydrous tetrahydrofuran is added 88 mg of 60% sodium hydride (in mineral oil). The mixture is refluxed for 2 hours and cooled. The mixture is mixed with alkyl iodide, stirred overnight, and mixed with water. The precipitating crystals are filtered off, washed with water, and with ethanol, and dried to give compound F as yellow crystals.

Compounds produced are shown in the following Table.

| R | amount of RI (mg) | yield (mg) | yield (%) | m.p. (°C) |
|---|---|---|---|---|
| Me | 300 | 452 | 76 | 253-258 (d) |
| Et | 600 | 390 | 65 | 216-218 (d) |
| n-Bu | 736 | 421 | 62 | 233-235 (d) |

Example 68

5-Methanesulfonyl-2-[3-(5-methylthienyl)]-2,5-dihydro-3H-pyrazolo[4,3-c]quinolin-3-one G

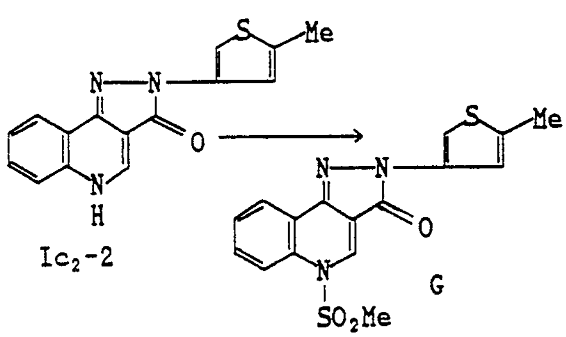

To a suspension of 2.86 g of 2-[3-(5-methylthienyl)]-2,5-dihydro-3H-pyrazolo[4,3-c]quinolin-3-one $Ic_2$-2 in 60 ml of anhydrous tetrahydrofuran is added 313 mg of 60% sodium hydride (mineral oil). The mixture is refluxed for 1 hour, cooled to 6°C, and to this mixture is dropwise added a solution of 1.26 g of methanesulfonyl chloride in 20 ml of anhydrous tetrahydrofuran. The mixture is stirred for 3.5 hours, concentrated under reduced pressure, mixed with water and acidified with acetic acid. The precipitating

material is collected by filtration, washed with water, dried, and recrystallized from chloroform-methanol to give 2.44 g (68%) of the orange crystalline compound G.

m.p.: 179—182°C (d)

| | | | | |
|---|---|---|---|---|
| Anal. Calcd. (%) (for $C_{16}H_{13}N_3O_3S_2$): | C, 53.46; | H, 3.64; | N, 11.69; | S, 17.84 |
| Found (%): | C, 53.31; | H, 3.85; | N, 11.56; | S, 17.43 |

Reference Example 1

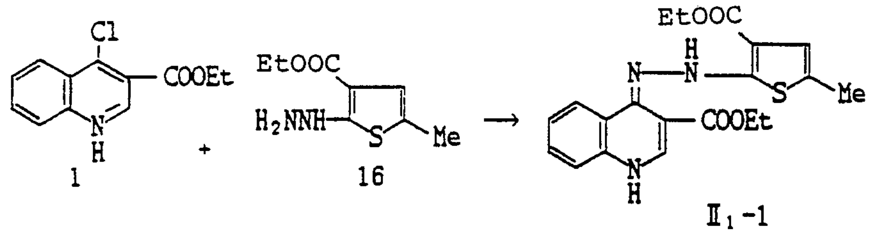

Ethyl 4-[2-(3-ethoxycarbonyl-5-methylthienyl)hydrazono]-1,4-dihydroquinoline-3-carboxylate $II_1$-1

To a solution of 942 mg of ethyl 4-chloroquinoline-3-carboxylate 1 in 10 ml of ethanol is added 880 mg of ethyl 2-hydrazino-5-methylthiophene-3-carboxylate 16. The mixture is stirred at room temperature for 1 hour and evaporated. The resulting residue is dissolved in chloroform and washed with cooled aqueous sodium bicarbonate and with water. The solution is dried over anhydrous magnesium sulfate and evaporated. The resulting solid is recrystallized from ethanol to give 1.47 g (yield: 92%) of the compound $II_2$-1 as orange crystals.

m.p.: 173—174°C

Reference Examples 2—13

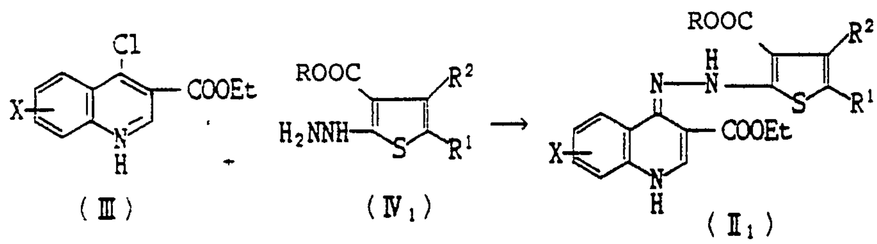

To a solution of an ethyl 4-chloroquinoline-3-carboxylate (III) in ethanol is added a 2-hydrazinothiophene-3-carboxylic acid ester ($IV_1$). The mixture is stirred at room temperature for 1 hour and evaporated. The resulting residue is dissolved in chloroform and washed with cold aqueous sodium bicarbonate and with water. The solution is dried over anhydrous magnesium sulfate and evaporated. The resulting solid is recrystallized from an appropriate solvent to give the compound ($II_1$) as orange crystals.

The reaction conditions to prepare compound ($II_1$) from compound (III) and compound ($IV_1$) as well as the physical properties of the resulting compound ($II_1$) are shown in Table 7.

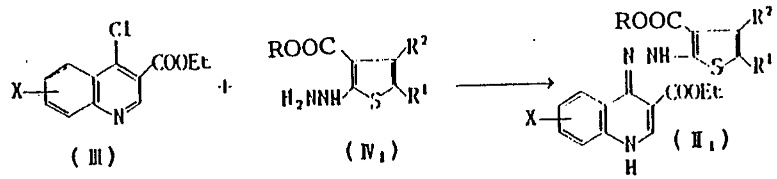

(III) (IV₁) (II₁)

Table 7

| Ref. Ex. No. | Amount (mg) Compd (III) | Compd. (IV₁) | Ethanol (ml) | Reaction tempera-ture (°C) | Reaction time (hrs) | Yield (g) | (%) | Compd. No. | $R^1$ | $R^2$ | R | X | R.S. | m.p. (°C) |
|---|---|---|---|---|---|---|---|---|---|---|---|---|---|---|
| 2 | 707 | 770 | 7 | reflux | 1 | 1.10 | 89 | $II_1$-2 | Et | H | Et | H | EtOH | 156-158 |
| 3 | 790 | 790 | 20 | 40 | 1 | 1.38 | 69 | $II_1$-3 | Me | Me | Et | H | " | 161-164 |
| 4 | 838 | 940 | 20 | 40 | 1 | 0.63 | 57 | $II_1$-4 | $-(CH_2)_4-$ | | Et | H | " | 176-177 |
| 5 | 1500 | 1600 | 15 | reflux | 0.5 | 2.55 | 91 | $II_1$-5 | n-Bu | H | Me | H | " | 173-175 |
| 6 | 749 | 614 | 15 | " | 1 | 1.11 | 93 | $II_1$-6 | Me | H | Me | 6-Me | " | 180-182 |
| 7 | 930 | 720 | 14 | " | 1.5 | 1.27 | 88 | $II_1$-7 | Me | H | Me | 6-MeO | " | 164-165 |
| 8 | 810 | 614 | 25 | " | 1.5 | 1.09 | 87 | $II_1$-8 | Me | H | Me | 6-Cl | " | 196-197 |
| 9 | 750 | 653 | 20 | " | 1.5 | 1.05 | 88 | $II_1$-9 | Me | H | Me | 6-F | " | 184-186 |
| 10 | 600 | 500 | 10 | " | 1 | 0.80 | 83 | $II_1$-10 | Me | H | Me | 7-Me | EtOH hexane | 169-170 |
| 11 | 1350 | 1030 | 23 | " | 1 | 1.83 | 88 | $II_1$-11 | Me | H | Me | 7-Cl | " | 186-187 |
| 12 | 707 | 899 | 20 | 50 | 0.5 | 1.29 | 91 | $II_1$-12 | COOEt | Me | Et | H | EtOH | 221-224(d) |
| 13 | 1330 | 1530 | 30 | r.t. | 0.9 | 2.34 | 91 | $II_1$-13 | H | COOEt | Et | H | " | 178-181(d) |

* r.t.: room temperature
** R.S.: recrystallization solvent

Reference Example 14

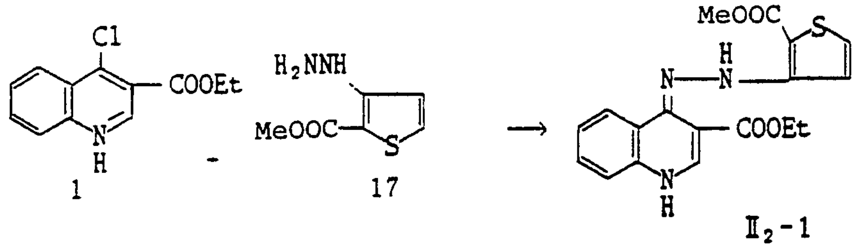

1 17 $II_2$-1

Ethyl 4-[3-(2-methoxycarbonylthienyl)hydrazono]-1,4-dihydroquinoline-3-carboxylate $II_2$-1

To a solution of 942 mg of ethyl 4-chloroquinoline-3-carboxylate 1 in 8 ml of ethanol is added 758 mg of methyl 3-hydrazinothiophene-2-carboxylate 17. The mixture is stirred at room temperature for 1 hour and dried under reduced pressure. The residue is dissolved in chloroform and washed with cold aqueous sodium bicarbonate and with water. The solution is dried and evaporated to give a solid. This is recrystallized from ethanol to give 1.46 g of compound $II_2$-1 as pale yellowish crystals.

m.p.: 161—162°C

Reference Examples 15—23

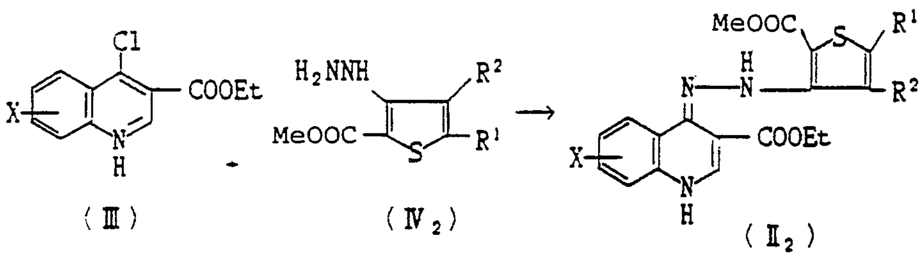

(III) ($IV_2$) ($II_2$)

To a solution of an ethyl 4-chloroquinoline-3-carboxylate (III) in ethanol is added a 3-hydrazinothiophene-2-carboxylic acid ester ($IV_2$). The mixture is stirred at room temperature for 1 hour and evaporated. The resulting residue is dissolved in chloroform and washed with cooled aqueous sodium bicarbonate and with water. The solution is dried over anhydrous magnesium sulfate and evaporated. The resulting solid is recrystallized from an appropriate solvent to give compound ($II_2$) as crystals.

The reaction conditions to prepare compound ($II_2$) from compound (III) and compound ($IV_2$), as well as the physical properties (m.p., appearance) of the resulting compound ($II_2$) are shown in Table 8.

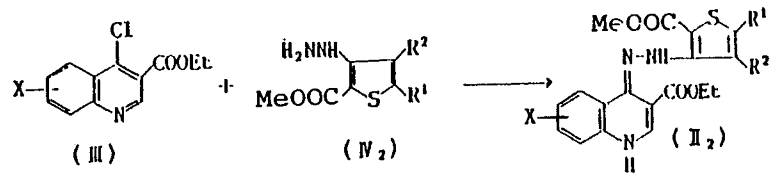

(III) (IV₂) (II₂)

Table 8

| Ref. Ex. No. | Amount (g) Compd.(III) | Amount (g) Compd.($IV_1$) | Ethanol (ml) | Reaction temperature (°C) | Reaction time (hrs) | Yield (g) | Yield (%) | Compound ($II_2$) Compd. No. | R¹ | R² | X | R. S. | Appearance | m.p. (°C) |
|---|---|---|---|---|---|---|---|---|---|---|---|---|---|---|
| 15 | 8.20 | 7.12 | 80 | r.t. | 1 | 12.90 | 96 | $II_1$-2 | Me | H | H | EtOH | pale yellow | 200 201 |
| 16 | 0.471 | 0.39 | 10 | 40 | 1 | 0.738 | 96 | $II_1$-3 | H | Me | H | " | yellow | 188-191 |
| 17 | 0.749 | 0.614 | 23 | r.t. | 1 | 1.08 | 90 | $II_1$-4 | Me | H | 6-Me | " | " | 197-200 |
| 18 | 0.93 | 0.716 | 15 | " | 1 | 1.28 | 88 | $II_1$-5 | Me | H | 6-MeO | " | " | 167-168 |
| 19 | 0.450 | 0.343 | 10 | 40 | 0.7 | 0.642 | 92 | $II_1$-6 | Me | H | 6-Cl | " | pale yellow | 222 223 |
| 20 | 0.75 | 0.653 | 25 | r.t. | 1 | 1.14 | 93 | $II_1$-7 | Me | H | 6-F | " | " | 198-201 |
| 21 | 0.87 | 0.722 | 15 | " | 1 | 1.22 | 88 | $II_1$-8 | Me | H | 7-Me | " | " | 176-178 |
| 22 | 1.08 | 0.82 | 20 | " | 1 | 1.59 | 95 | $II_1$-9 | Me | H | 7-Cl | " | yellow | 198-199 |
| 23 | 0.50 | 0.445 | 5 | " | 1 | 0.71 | 84 | $II_2$-10 | Et | H | H | " | red | 166-168 |

* r.t.: room temperature
** R.S.: recrystallization solvent

Reference Example 24

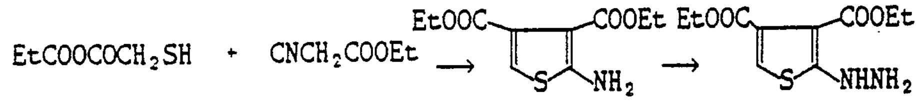

Diethyl 2-hydrazinothiophene-3,4-dicarboxylate H

To a solution of 14.3 g of ethyl mercaptopyruvate in 60 ml of ethanol is added 11.5 g of ethyl cyanoacetate at 5°C. The mixture is mixed with 0.7 ml of piperidine, stirred at room temperature for 50 minutes, and evaporated. The residue is dissolved in ethyl acetate, washed with water, and evaporated. The resulting residue is crystallized from ether-hexane to give 20.2 g (86%) of diethyl 2-aminothiophene-3,4-carboxylate as crystals, melting at 115—118°C.

To a mixture of the crystals (4.86 g) and 30 ml of conc. hydrochloric acid is dropwise added 15 ml of an aqueous solution of 1.52 g of sodium nitrite at −10 ~ 5°C; and the mixture is stirred at 15°C for 30 minutes to give a solution of a diazonium salt. A solution of 26 g of stannous chloride in 40 ml of conc. hydrochloric acid is cooled below 5°C in another flask, and the solution of the diazonium salt is added thereto with stirring. The mixture is stirred at 0—5°C for 30 minutes; and the precipitating crystals are collected by filtration and washed with ether. The crystals are mixed with ethyl acetate, basified with 2N sodium hydroxide, and extracted with ethyl acetate. The extract is washed with water, dried, and evaporated. The resulting residue is purified by silica-gel column chromatography and the fractions eluted with methylene chloride are concentrated to give 1.96 g (38%) of the compound H as crystals.

m.p.: 53.5—55°C

Preparation

| | |
|---|---|
| 2-[3-(5-Chlorothienyl)]-2,5-dihydro-3H-pyrazolo[4,3-c]quinolin-3-one | 100 mg |
| wheat starch | 48 mg |
| magnesium stearate | 2 mg |

The above components are mixed each other to prepare a capsule.

## Claims

1. A pyrazolo[4,3-c]quinolin-3-one derivative represented by the formula:

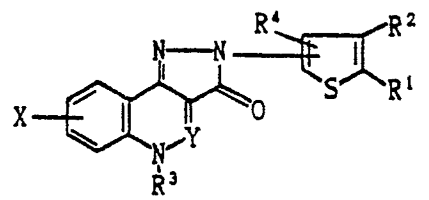

(I)

wherein $R^1$ and $R^2$ each are hydrogen, $(C_1—C_5)$alkyl, $(C_2—C_5)$alkoxycarbonyl, carboxyl, halogen, nitro or trifluoromethyl, or $R^1$ and $R^2$ when taken together are $(C_3—C_4)$alkylene; $R^3$ is hydrogen, $(C_1—C_5)$alkyl, $(C_1—C_5)$alkanoyl or $(C_1—C_5)$alkylsulfonyl; $R^4$ is hydrogen, $(C_2—C_6)$alkoxycarbonyl, carboxyl, or halogen; X is hydrogen, $(C_1—C_5)$alkyl, $(C_1—C_5)$alkoxy, halogen or hydroxyl; and Y is methine or nitrogen; or a salt thereof.

2. A compound as claimed in claim 1, wherein Y is methine.

3. A compound as claimed in claim 1, wherein $R^1$ and $R^2$ each are hydrogen or $(C_1—C_5)$alkyl.

4. A compound as claimed in claim 1, wherein X is hydrogen, $(C_1—C_5)$alkyl, $(C_1—C_5)$alkoxy or halogen.

5. A compound as claimed in claim 1, wherein $R^1$ and $R^2$ each are hydrogen or $(C_1—C_5)$alkyl; $R^3$ is hydrogen; $R^4$ is hydrogen; X is hydrogen or $(C_1—C_5)$alkyl; and Y is methine.

6. Use of a compound as claimed in claim 1, for preparing a pharmaceutical composition having psychotropic activity.

**Patentansprüche**

1. Pyrazolo[4,3-c]chinolin-3-on-derivat der Formel:

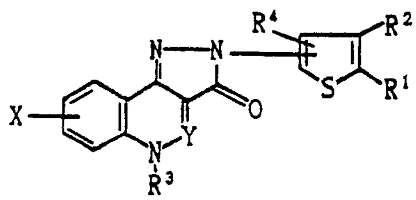

(I)

worin $R^1$ und $R^2$ je Wasserstoff, $(C_1—C_5)$-Alkyl, $(C_2—C_5)$-Alkoxycarbonyl, Carboxyl, Halogen, Nitro oder Trifluormethyl bedeuten oder worin $R^1$ und $R^2$ zusammen $(C_3—C_4)$-Alkylen bedeuten, $R^3$ Wasserstoff, $(C_1—C_5)$-Alkyl, $(C_1—C_5)$-Alkanoyl oder $(C_1—C_5)$-Alkylsulfonyl bedeutet, $R^4$ Wasserstoff, $(C_2—C_6)$-Alkoxycarbonyl, Carboxyl oder Halogen bedeutet, X Wasserstoff, $(C_1—C_5)$-Akyl, $(C_1—C_5)$-Alkoxy, Halogen oder Hydroxyl bedeutet und Y Methin oder Sticktoff bedeutet, oder eines seiner Salze.

2. Verbindung nach Anspruch 1, worin Y Methin bedeutet.

3. Verbindung nach Anspruch 1, worin $R^1$ und $R^2$ je Wasserstoff oder $(C_1—C_5)$-Alkyl bedeuten.

4. Verbindung nach Anspruch 1, worin X Wasserstoff $(C_1—C_5)$-Alkyl, $(C_1—C_5)$-Alkoxy oder Halogen bedeutet.

5. Verbindung nach Anspruch 1, worin $R^1$ und $R^2$ je Wasserstoff, $(C_1—C_5)$-Alkyl bedeuten, $R^3$ Wasserstoff, $R^4$ Wasserstoff, X Wasserstoff oder $(C_1—C_5)$-Alkyl und Y Methin bedeuten.

6. Verwendung einer Verbindung nach Anspruch 1 für die Herstellung eines pharmazeutischen Präparats mit psychotropischer Aktivität.

**Revendications**

1. Dérivé de pyrazolo[4,3-c]quinolinone-3 représenté par la formule:

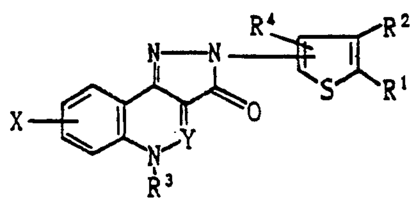

(I)

dans laquelle:

$R^1$ et $R^2$ représentent chacun hydrogène, alkyle en $C_1—C_5$, alcoxycarbonyle en $C_2—C_5$, carboxyle halogène, nitro ou trifluorométhyle, ou bien $R^1$ et $R^2$, lorsqu'ils sont pris ensemble, représentent alkylène en $C_3—C_4$;

$R^3$ représente hydrogène, alkyle en $C_1—C_5$, alcanoyle en $C_1—C_5$ ou alkylsulfonyle en $C_1—C_5$;

$R^4$ représente hydrogène, alcoxycarbonyle en $C_2—C_6$, carboxyle, ou halogène;

X représente hydrogène, alkyle en $C_1—C_5$, alcoxy en $C_1—C_5$, halogène ou hydroxyle; et

Y représente méthine ou azote,

ou sel de ce dérivé.

2. Composé selon la revendication 1, dans lequel Y représente méthine.

3. Composé selon la revendication 1, dans lequel $R^1$ et $R^2$ représentent chacun hydrogène ou alkyle en $C_1—C_5$.

4. Composé selon la revendication 1, dans lequel X représente hydrogène, alkyle en $C_1—C_5$, alcoxy en $C_1—C_5$ ou halogène.

5. Composé selon la revendication 1, dans lequel $R^1$ et $R^2$ représentent chacun hydrogène ou alkyle en $C_1—C_5$; $R^3$ représente hydrogène; $R^4$ représente hydrogène; X représente hydrogène ou alkyle en $C_1—C_5$; et Y représente méthine.

6. Utilisation d'un composé selon la revendication 1, pour la préparation d'une composition pharmaceutique ayant une activité psychotrope.